# EUROPÄISCHE PATENTSCHRIFT

(11) **EP 4 146 312 B1**
(45) Veröffentlichungstag und Bekanntmachung des Hinweises auf die Patenterteilung: **01.05.2024**
(21) Anmeldenummer: 21725080.2
(22) Anmeldetag: 05.05.2021
(51) Int. Cl.: A61M 16/00, A61B 5/08

(54) **BEATMUNGSVORRICHTUNG, WELCHE ZUR ERMITTLUNG EINER FUNKTIONELLEN BEEINTRÄCHTIGUNG IHRER O2-SENSORANORDNUNG AUSGEBILDET IST**
VENTILATION DEVICE DESIGNED TO IDENTIFY FUNCTIONAL IMPAIRMENT OF ITS O2 SENSOR ASSEMBLY
DISPOSITIF DE VENTILATION CONÇU POUR IDENTIFIER UNE DÉFICIENCE FONCTIONNELLE DE SON ENSEMBLE CAPTEUR D'O2

(30) Priorität: 08.05.2020 DE 102020112557
(43) Veröffentlichungstag der Anmeldung: 15.03.2023
(73) Patentinhaber: Hamilton Medical AG, 7402 Bonaduz (CH)
(72) Erfinder: SCHRANZ, Christoph, 7306 Fläsch (CH); SCHAD, Jonathan, 7402 Bonaduz (CH); NOVOTNI, Dominik, 7000 Chur (CH); OFFENBECK, Bernd, 93057 Regensburg (DE)
(74) Vertreter: Ruttensperger Lachnit Trossin Gomoll
(86) Internationale Anmeldenummer: PCT/EP2021/061874
(87) Internationale Veröffentlichungsnummer: WO 2021/224333

(56) Entgegenhaltungen:
- WO-A1-2019/094680
- WO-A1-2021/009215
- DE-A1-102017 204 082
- DE-A1-102017 217 859
- DE-A1-102018 207 666
- US-B2- 7 833 480

## Beschreibung

Die vorliegende Erfindung betrifft eine Beatmungsvorrichtung zur künstlichen Beatmung gemäß dem Oberbegriff von Anspruch 1. Diese Beatmungsvorrichtung umfasst
- einer Beatmungsgasquelle,
- einer Beatmungsleitungsanordnung, um inspiratorisches Beatmungsgas von der Beatmungsgasquelle zu einer patientenseitigen, proximalen Beatmungsgas-Auslassöffnung zu leiten und um exspiratorisches Beatmungsgas von einer proximalen Beatmungsgas-Einlassöffnung weg zu leiten,
- einer Druckveränderungsanordnung zur Veränderung des Drucks des in der Beatmungsleitungsanordnung strömenden Beatmungsgases,
- einer Steuervorrichtung, welche dazu ausgebildet ist, den Betrieb der Beatmungsgasquelle oder/und der Druckveränderungsanordnung zu steuern,
- einer Auswertevorrichtung zur Verarbeitung von Sensorsignalen, und
- einer O₂-Sensoranordnung zur Ermittlung eines einen Sauerstoffgehalt des in der Beatmungsleitungsanordnung strömenden Beatmungsgases repräsentierenden O₂-Gehaltswerts, wobei die O₂-Sensoranordnung O₂-Sensorsignale, welche Information über den O₂-Gehaltswert enthalten, an die Auswertevorrichtung ausgibt.

Eine derartige Beatmungsvorrichtung zur künstlichen Beatmung mit einer Beatmungsgasquelle, einer Beatmungsleitungsanordnung, einer Druckveränderungsanordnung und einer Steuervorrichtung ist aus der DE 10 2017 204 082 A1 bekannt. Aus dieser Druckschrift ist außerdem eine O₂-Sensoranordnung bekannt, welche nach dem Prinzip der Lumineszenzlöschung arbeitet. Dabei wird ein luminophorhaltiges Material durch eine Strahlungsquelle zum Leuchten angeregt. Das durch eine Erfassungsvorrichtung erfasste angeregte Leuchtverhalten ist dabei abhängig von der Menge an Sauerstoff, die als Bestandteil des Beatmungsgases in Kontakt mit dem angeregten Luminophor gelangt. In Kontakt mit dem angeregten Luminophor gelangende Sauerstoffmoleküle entregen den Luminophor und vermindern so dessen Leuchtintensität oder/und -dauer. Je größer der Sauerstoffanteil am Beatmungsgas ist, desto stärker fällt die Entregung des Luminophors aus.

Aus der DE 10 2018 207 666 A1 ist ein weiterer nach dem Prinzip der Lumineszenzlöschung arbeitender O₂-Sensor bekannt, welcher zum Einsatz an einer Beatmungsleitungsanordnung ausgebildet ist, um einen Sauerstoffgehalt des in der Beatmungsleitungsanordnung strömenden Beatmungsgases zu ermitteln.

Außerdem weisen Beatmungsvorrichtungen häufig eine CO₂-Sensoranordnung auf, um im Beatmungsgas enthaltenes CO₂ zu erfassen. Zur Ermittlung eines CO₂-Anteils eines Beatmungsgases sind infrarotspektroskopische Sensoranordnungen bekannt, welche einen Beatmungsgasstrom mit Infrarotlicht durchleuchten und aufgrund der Absorption von Infrarotlicht durch Kohlenstoffdioxid in an sich bekannter Weise die Ermittlung des Kohlenstoffdioxidgehalts im Beatmungsgas gestatten. Solche kapnometrischen Infrarot-Sensoranordnungen sind beispielsweise bekannt aus der US 6,095,986 A, der GB 2 533 806 B und der DE 10 2006 052 999 A1.

Das Beatmungsgas weist eine gewisse Feuchte auf, die sich bei unvorteilhaften thermischen Verhältnissen an Festkörperoberflächen in der Beatmungsleitungsanordnung und ebenso in den von Beatmungsgas durchströmten Sensoranordnungen als Flüssigkeit niederschlagen kann. Dies gilt sowohl für inspiratorisches Beatmungsgas, das hinsichtlich seines Feuchtegrades konditioniert wird, damit es für den Patienten möglichst verträglich ist, als auch für exspiratorisches Beatmungsgas, das zusätzlich um Körperflüssigkeiten angereichert sein kann, die vom Patienten über den Atemweg abgegeben werden, wie etwa Speichel und Schleim.

Während sich niederschlagende Feuchtigkeit die Messgenauigkeit infrarotspektroskopischer CO₂-Sensoranordnungen häufig nicht besonders beeinträchtigt bzw. eine etwaige Beeinträchtigung ohne weiteres kompensiert werden kann, stellt Feuchtigkeitsniederschlag für O₂-Sensoranordnungen ein erhebliches Problem dar. Da die Ermittlungen des Kohlenstoffdioxidgehalts und des Sauerstoffgehalts im Beatmungsgas zur Beurteilung der Verstoffwechselung des Beatmungsgases durch den Patienten herangezogen werden und folglich Einfluss auf die Beatmungsparameter der künstlichen Beatmung haben können, ist die Ermittlung des Kohlenstoffdioxidgehalts und des Sauerstoffgehalts im Beatmungsgas mit möglichst hoher Ermittlungsgenauigkeit von besonderem Interesse.

Aus der US 7,833,480 B2 ist eine weitere nach dem Prinzip der Lumineszenzlöschung arbeitende O₂-Sensoranordnung bekannt. In dieser Druckschrift ist die Empfindlichkeit der bekannten O₂-Sensoranordnung gegenüber Feuchtigkeit erwähnt. Um dem Einfluss von Feuchtigkeit auf die Funktion der bekannten O₂-Sensoranordnung entgegenzutreten, erwähnt die US 7,833,480 B2 lediglich allgemein die Verwendung von Kompensationsalgorithmen, um durch Feuchtigkeit bewirkte Messfehler zu kompensieren. Die Kompensationsalgorithmen selbst sind in der Druckschrift weder durch unmittelbare Erläuterung noch durch Verweis auf entsprechende Quellen näher ausgeführt. Denkbar ist, dass die aus der US 7,833,480 B2 bekannte Vorrichtung einen Feuchtigkeitssensor verwendet, um abhängig von dessen Signal die O₂-Sensorsignale der bekannten O₂-Sensoranordnung zu kompensieren.

Aufgabe der vorliegenden Erfindung ist es daher, eine Beatmungsvorrichtung anzugeben, welche nicht nur in der Lage ist, einen Sauerstoffgehalt im Beatmungsgas zu ermitteln, sondern welche auch dazu in der Lage ist, die zur Ermittlung des Sauerstoffgehalts vorgesehene O₂-Sensoranordnung auf Beeinträchtigungen durch sich niederschlagende Feuchtigkeit zu überprüfen und erforderlichenfalls einen Benutzer der Beatmungsvorrichtung entsprechend zu informieren.

Diese Aufgabe wird gemäß der vorliegenden Erfindung gelöst durch eine Beatmungsvorrichtung, wie sie eingangs angegeben ist, bei welcher die Auswertevorrichtung dazu ausgebildet ist, auf Grundlage der O₂-Sensorsignale einen eine Änderung des O₂-Gehaltswerts repräsentierenden O₂-Änderungswert zu bestimmen und dann, wenn der O₂-Änderungswert eine vorbestimmte Bedingung erfüllt, auf eine Degradation der O₂-Sensoranordnung zu schließen und ein Signal auszugeben.

Bevorzugt ist die O₂-Sensoranordnung eine nach dem Prinzip der Lumineszenzlöschung arbeitende Sensoranordnung. Derartige Sensoranordnungen und ihre Arbeitsweise sind im oben genannten Stand der Technik ausführlich beschrieben und werden als bekannt vorausgesetzt. Die O₂-Sensoranordnung kann jedoch eine beliebige andere auf Kontakt von Sauerstoffmolekülen mit einer Reaktionssubstanz oder/und einer Katalysatorsubstanz beruhende O₂-Sensoranordnung sein. Lediglich zur Veranschaulichung wird nachfolgend zur Beschreibung der Erfindung das Beispiel einer nach dem Prinzip der Lumineszenzlöschung arbeitenden Sensoranordnung herangezogen.

Der zur Ermittlung des Sauerstoffgehalts beobachtbare, mit einem anregbaren Luminophor ausgerüstete Observationsbereich einer solchen O₂-Sensoranordnung in einer Beatmungsleitungsanordnung ist in groben Zügen wie folgt aufgebaut: zum Strömungsraum des Beatmungsgases hin ist eine nachfolgend als "Membran" bezeichnete sauerstoffdurchlässige Trägersubstratschicht angeordnet, welche einen zur Lumineszenz anregbaren Luminophor trägt. Der Luminophor kann in ein als Matrix wirkendes Material des Trägersubstrats eingebettet sein oder kann auf der vom Strömungsraum des Beatmungsgases abgewandten Seite als Luminophorschicht aufgetragen sein. Da das Beatmungsgas die ihm zugewandte Seite der sauerstoffdurchlässigen Membran benetzen kann, kann im Beatmungsgas enthaltener Sauerstoff den Luminophor der O₂-Sensoranordnung erreichen und dessen angeregte Lumineszenz ablöschen.

Auf der vom Strömungsraum des Beatmungsgases abgewandten Seite ist der Luminophor durch eine sauerstoffdichte Deckschicht abgedeckt, um zu verhindern, dass Sauerstoff der Umgebungsluft zur Ablöschung der angeregten Lumineszenz beiträgt und so das Ergebnis verfälscht, das eine Aussage nur über den Sauerstoffgehalt des Beatmungsgases geben soll.

Vom Beatmungsgas mitgeführte Feuchtigkeit oder/und Flüssigkeit, die sich auf der dem Strömungsraum zugewandten Seite des Observationsbereichs niederschlägt, verhindert oder behindert, dass der im Beatmungsgas enthaltene Sauerstoff den Luminophor in einem dem Sauerstoffanteil am Beatmungsgas entsprechendem Ausmaß erreicht und folglich eine seinem Anteil am Beatmungsgas entsprechende Löschung der angeregten Lumineszenz des Luminophors bewirkt.

Dabei kann ein Flüssigkeitsfilm die Membran zum Strömungsraum hin teilweise oder sogar vollständig bedecken oder/und kann auf der Membran niedergeschlagene Flüssigkeit aufgrund von Kapillarwirkung in die sauerstoffdurchlässige Membran migrieren und dadurch deren Porosität teilweise oder vollständig ausfüllen. Im Ergebnis wird von einer derart flüssigkeitsbelasteten O₂-Sensoranordnung ein niedrigerer Sauerstoffgehalt des Beatmungsgases als der tatsächliche Sauerstoffgehalt ermittelt, und zwar umso niedriger je stärker die Belastung durch Flüssigkeit ist. Genauer wird aufgrund der unerwünschten Flüssigkeitsbelastung eine Änderung des Sauerstoffgehalts als langsamer erfasst als sie sich tatsächlich vollzieht. Über eine begrenzte Erfassungsdauer, wie etwa einer Inspirationsphase oder einer Exspirationsphase, hinweg resultiert die langsamer als tatsächlich erfasste Änderung des Sauerstoffgehalts in einem O₂-Gehaltswert, der einen betragsmäßig niedrigeren Sauerstoffgehalt als den tatsächlichen repräsentiert.

Da der Sauerstoff in der Patientenlunge zu Kohlenstoffdioxid verstoffwechselt wird, ist der Kohlenstoffdioxidgehalt im exspiratorischen Beatmungsgas größer als im inspiratorischen Beatmungsgas und ist der Sauerstoffgehalt im inspiratorischen Beatmungsgas größer als im exspiratorischen Beatmungsgas. Somit ändert sich zum einen sowohl der Kohlenstoffdioxidgehalt als auch der Sauerstoffgehalt des Beatmungsgases zwischen den Atemhub-Teilphasen: Inspirationsphase und Exspirationsphase. Zum anderen ändert sich auch innerhalb einer Atemhub-Teilphase ein O₂-Gehaltswert und ändert sich auch innerhalb einer Atemhub-Teilphase, insbesondere innerhalb einer Exspirationsphase, ein CO₂-Gehaltswert.

In Sinne der vorliegenden Anmeldung umfasst ein Atemhub eine Exspirationsphase und eine Inspirationsphase, wobei die eine Phase unmittelbar auf die andere folgt. Dabei kommt es auf die Reihenfolge der Phasen nicht an.

Problematisch an der vorliegenden Erfassungssituation ist, dass die O₂-Sensoranordnung stets O₂-Sensorsignale liefert, von denen aber zunächst nicht klar ist, ob sie Ergebnis einer korrekten Erfassung sind. Die vorliegende Erfindung stellt zur Beurteilung einer Beeinträchtigung der O₂-Sensoranordnung nicht auf die O₂-Sensorsignale selbst oder auf den durch sie jeweils repräsentierten O₂-Gehaltswert ab, sondem zieht hierzu das Änderungsverhalten der O₂-Sensorsignale und des durch sie repräsentierten O₂-Gehaltswerts heran. Aus dem Änderungsverhalten lassen sich auf unterschiedliche Weisen Informationen über eine Flüssigkeitsbelastung der O₂-Sensoranordnung erhalten.

Um eine fehlerhafte Ausgabe eines eine Degradation der O₂-Sensoranordnung anzeigenden Signals durch die Auswertevorrichtung möglichst zu verhindern, ist die Auswertevorrichtung bevorzugt dazu ausgebildet ist, das Signal nur dann auszugeben, wenn die vorbestimmte Bedingung eine vorbestimmte Mehrzahl von Malen innerhalb einer vorbestimmten Mehrzahl von Atemhüben erfüllt ist. Dies ermöglicht eine sichere Beurteilung einer Beeinträchtigung der O₂-Sensoranordnung in einer immer noch sehr kurzen Zeit. Beispielsweise kann das Signal erst dann ausgegeben werden, wenn die Bedingung drei Mal über wenigstens drei Atemhübe hinweg erfüllt ist.

Bevorzugt wird für einen Atemhub genau ein Prüfvorgang zur Überprüfung der Funktionstüchtigkeit der O₂-Sensoranordnung ausgeführt. Deshalb ist bevorzugt die Anzahl von erforderlichen Erfüllungsereignissen der Bedingung gleich der vorbestimmten Mehrzahl an Atemhüben.

Für eine möglichst schnelle Erfassung einer Beeinträchtigung der O₂-Sensoranordnung ist bevorzugt eine jeweilige Erfüllung der Bedingung über nicht mehr als zehn Atemhübe vorteilhaft. In Versuchen hat sich eine Anzahl von fünfmaliger Erfüllung der vorbestimmten Bedingung über fünf Atemhübe hinweg als sehr guter Kompromiss zwischen genauer und schneller Erfassung der Beeinträchtigung der O₂-Sensoranordnung besonders bewährt. Um die Zeitdauer bis zu einem Erkennen der Beeinträchtigung möglichst kurz zu halten, folgen die Atemhübe der Mehrzahl von Atemhüben unmittelbar aufeinander, wenngleich dies nicht zwingend notwendig ist.

Wie unten näher erläutert wird, kann in einem Prüfvorgang zur Ermittlung einer Beeinträchtigung der O₂-Sensoranordnung durch die Auswertevorrichtung genau eine vorbestimmte Bedingung oder auch eine Mehrzahl von unterschiedlichen vorbestimmten Bedingungen abgeprüft werden, etwa zwei vorbestimmte Bedingungen, nämlich das Unterschiedskriterium der ersten Ausführungsform und das Gradientenkriterium der zweiten Ausführungsform. Dabei reicht es jedoch bevorzugt aus, wenn nur genau eine der abgeprüften Bedingungen erfüllt ist.

In gleicher Weise kann die Auswertevorrichtung dazu ausgebildet sein, nach Ausgabe eines die Beeinträchtigung der O₂-Sensoranordnung anzeigenden Signals ein die Aufhebung der Beeinträchtigung anzeigendes weiteres Signal dann auszugeben, wenn die vorbestimmte Bedingung oder, im Falle einer Mehrzahl von abgeprüften Bedingungen, wenn jede Bedingung aus einer Mehrzahl von Bedingungen eine vorbestimmte Mehrzahl von Malen innerhalb einer vorbestimmten Mehrzahl von Atemhüben nicht erfüllt ist.

Die Auswertevorrichtung kann durch die Steuervorrichtung oder einen Teil der Steuervorrichtung gebildet sein. Bevorzugt umfasst die Auswertevorrichtung oder/und die Steuervorrichtung einen integrierten Schaltkreis zur Ausführung von datenverarbeitenden Operationen und einen Datenspeicher zur abrufbaren Speicherung von zu verarbeitenden Daten. Die Auswertevorrichtung kann vollständig oder wenigstens zum Teil an wenigstens einer der Sensoranordnungen lokalisiert sein, denn auch im Gehäuse einer Sensoranordnung können elektronische Schaltkreise zur Signalspeicherung und Signalverarbeitung des jeweiligen wenigstens einen erfassenden Sensorelements der Sensoranordnung angeordnet sein. Zur Sicherstellung einer Bereitstellung oder/und Ausgabe möglichst definierter Sensorinformation, ist bevorzugt wenigstens jeweils ein Teil der Auswertevorrichtung bei der O₂-Sensoranordnung, ebenso wie bei der weiter unten genannten CO₂-Sensoranordnung, angeordnet. Diese Auswertevorrichtung oder Teil- Auswertevorrichtung verarbeitet Sensorsignale der jeweils zugeordneten Sensoranordnung und gibt verarbeitete oder vorverarbeitete Sensorsignale an einen weiteren Teil der Auswertevorrichtung, sofern vorhanden, oder an die Steuervorrichtung aus.

Gemäß einer ersten Ausführungsform der vorliegenden Erfindung kann die Auswertevorrichtung dazu ausgebildet sein, auf Grundlage der O₂-Sensorsignale einen O₂-Unterschiedswert als den O₂-Änderungswert zu ermitteln. Dieser O₂-Unterschiedswert repräsentiert einen betragsmäßigen Unterschied zwischen einem charakteristischen exspiratorischen O₂-Gehaltswert des exspiratorischen Beatmungsgases und einem charakteristischen inspiratorischen O₂-Gehaltswert des inspiratorischen Beatmungsgases. Die vorbestimmte Bedingung ist, dass der O₂-Unterschiedswert in einer vorbestimmten relativen Beziehung zu einem O₂-Unterschiedsgrenzwert steht. Diese Bedingung und ihre Weiterbildungen sind in der vorliegenden Anmeldung als "Unterschiedskriterium" bezeichnet.

Mit "vorbestimmter relativer Beziehung" ist eine Vergleichsbeziehung wie "größer als", "kleiner als", "größer gleich" und "kleiner gleich" bezeichnet. Die konkret angemessene relative Vergleichsbeziehung hängt von der konkret verwendeten Definition des O₂-Unterschiedswerts ab.

Mit dem Attribut "charakteristisch" ist lediglich ausgesagt, dass der so bezeichnete charakteristische O₂-Gehaltswert nach vorbestimmten Regeln aus einer Mehrzahl von vorhandenen O₂-Gehaltswerten eines Atemhubs oder einer Atemhub-Teilphase ausgewählt oder bestimmt oder berechnet wird.

Ein eine Beeinträchtigung der O₂-Sensoranordnung anzeigendes Ansprechverhalten kommt gemäß dem Unterschiedskriterium der ersten Ausführungsform bevorzugt darin zum Ausdruck, dass der Betrag des O₂-Unterschiedswerts einen vorbestimmten O₂-Unterschiedsgrenzwert nicht übersteigt. Dieses Unterschiedskriterium basiert auf der Erkenntnis, dass eine flüssigkeitsbelastete O₂-Sensoranordnung zwar nach wie vor einen Sauerstoffgehalt im Beatmungsgas erfasst, dieser jedoch voraussichtlich betragsmäßig zu niedrig ist. Das Verhältnis der ermittelten Sauerstoffgehalte von exspiratorischem und inspiratorischem Beatmungsgas zueinander kann dabei von der Beeinträchtigung nahezu unbeeinflusst sein. Jedoch nimmt bei ausreichender Beeinträchtigung der O₂-Sensoranordnung der betragsmäßige Abstand zwischen dem Sauerstoffgehalt des inspiratorischen Beatmungsgases und dem Sauerstoffgehalt des exspiratorischen Beatmungsgases ab. Das Unterschiedskriterium auf Grundlage des O₂-Unterschiedswert reflektiert diesen Umstand, so dass die O₂-Sensoranordnung als degradiert bzw. beeinträchtigt gilt, wenn der betragsmäßige Abstand zwischen dem Sauerstoffgehalt des inspiratorischen Beatmungsgases und dem Sauerstoffgehalt des exspiratorischen Beatmungsgases betragsmäßig zu gering ist.

Je nachdem, ob die vorzeichenlosen Beträge von Unterschiedswerten oder die vorzeichenbehafteten Unterschiedswerte betrachtet werden, ändert sich bei negativen Unterschiedswerten im Vergleich zur Verwendung nur ihrer Beträge die vorbestimmte relative Beziehung eines Unterschiedswerts zu dem ihm zugeordneten Unterschiedsgrenzwert.

Bei Anwendung des Unterschiedskriteriums kann die Genauigkeit der Beurteilung der Funktionstüchtigkeit der O₂-Sensoranordnung durch Berücksichtigung der Umgebungsatmosphäre erhöht werden. In der Regel gibt die O₂-Sensoranordnung O₂-Sensorsignale aus, welche ausgehend vom angewandten Messprinzip den Partialdruck von O₂ im Beatmungsgas als den jeweiligen O₂-Gehaltswert repräsentieren. In Kenntnis des Atmosphärendrucks als einem bevorzugten Beispiel eines Atmosphärenzustandswerts können die als Partialdrücke erhaltenen Gehaltswerte in ihrer Bedeutung zutreffender bewertet werden. Die Auswertevorrichtung ist daher bevorzugt dazu ausgebildet, bei einer Durchführung des Vergleichs eines O₂-Unterschiedswerts mit einem O₂-Unterschiedsgrenzwert wenigstens einen Atmosphärenzustandswert zu berücksichtigen, welcher einen Zustand der Umgebungsatmosphäre der Beatmungsvorrichtung repräsentiert.

Diese Berücksichtigung des Atmosphärenzustandswerts, bevorzugt des Atmosphärendrucks, kann durch die Auswertevorrichtung gemäß einer ersten Alternative dadurch erfolgen, dass die Auswertevorrichtung den O₂-Unterschiedsgrenzwert in Abhängigkeit von dem wenigstens einen Atmosphärenzustandswert ermittelt. Beispielsweise kann die Auswertevorrichtung als einen den Atmosphärenzustand berücksichtigenden O₂-Unterschiedsgrenzwert das Produkt aus O₂-Unterschiedsgrenzwert und Atmosphärenzustandswert bilden.

Gemäß einer Alternative kann die Auswertevorrichtung den Atmosphärenzustandswert dadurch berücksichtigen, dass die Auswertevorrichtung aus dem O₂-Unterschiedswert und dem wenigstens einen Atmosphärenzustandswert einen atmosphärenbezogenen O₂-Unterschiedswert ermittelt. Beispielsweise kann die Auswertevorrichtung als atmosphärenbezogenen O₂-Unterschiedswert das Verhältnis des O₂-Unterschiedswerts zu dem wenigstens einen Atmosphärenzustandswert bilden.

In der Beatmungstechnik haben sich Drücke als Zustandswerte von Gasen bewährt, weshalb bevorzugt der wenigstens eine Atmosphärenzustandswert den Atmosphärendruck repräsentiert, der O₂-Unterschiedswert einen O₂-Partialdruck-Unterschiedswert zwischen einem O₂-Partialdruck in dem exspiratorischen Beatmungsgas und einem O₂-Partialdruck in dem inspiratorischen Beatmungsgas repräsentiert. Wie oben bereits dargelegt, können mit den physikalischen Wirkprinzipien, auf welchen die verfügbaren O₂-Sensoranordnungen in ihrer Wirkungsweise beruhen, partialdruck-proportionale O₂-Sensorsignale unmittelbar erhalten werden.

Ein atmosphärenbezogener Unterschiedswert muss jedoch nicht aus gleichen physikalischen Größen gebildet sein, sondern kann ein bloßer Zahlenwert sein, der durch mathematische Verknüpfung unterschiedlicher physikalischer Größen gebildet wird.

Um bei Anwendung des Unterschiedskriteriums möglichst aussagekräftige O₂-Gehaltswerte zu verwenden, sollten diese nicht zu nah am Beginn oder am Ende ihrer Atemhub-Teilphase bestimmt werden. Daher entstammt der charakteristische inspiratorische O₂-Gehaltswert bevorzugt einem Inspirationsphasenabschnitt, welcher einen ersten zeitlichen Abstand vom Beginn einer Inspirationsphase und einen zweiten zeitlichen Abstand vom Ende der Inspirationsphase entfernt gelegen ist. Zusätzlich oder alternativ entstammt der charakteristische exspiratorische O₂-Gehaltswert bevorzugt einem Exspirationsphasenabschnitt, welcher einen dritten zeitlichen Abstand vom Beginn einer Exspirationsphase und einen vierten zeitlichen Abstand vom Ende der Exspirationsphase entfernt gelegen ist.

Bevorzugt sind die ersten bis vierten zeitlichen Abstände so groß gewählt, dass die O₂-Gehaltswerte nicht in den transienten Abschnitten der jeweiligen Atemhub-Teilphase bestimmt werden, in welchen sich die O₂-Gehaltswerte in kurzen Zeitinkrementen betragsmäßig stark ändern, also beispielsweise nicht in den steigenden und fallenden Flanken einer den O₂-Gehalt über die Zeit angebenden O₂-Gehaltskurve.

Da die vorliegend beschriebene Bestimmung einer Beeinträchtigung der O₂-Sensoranordnung während des laufenden Beatmungsbetriebs durchgeführt wird, ist es vorteilhaft, wenn die Bestimmung der Funktionstüchtigkeit so wenig Systemressourcen der Beatmungsvorrichtung wie möglich in Anspruch nimmt. Eine Bestimmung in möglichst kurzer Zeit und mit möglichst geringem Aufwand an Rechenleistung kann beispielsweise dadurch unterstützt werden, dass die Auswertevorrichtung dazu ausgebildet ist, den zuvor genannten Inspirationsphasenabschnitt oder/und den zuvor genannten Exspirationsphasenabschnitt zu identifizieren und nur darin einen charakteristischen O₂-Gehaltswert zu ermitteln. Somit muss der charakteristische O₂-Gehaltswert nicht über die gesamte Inspirationsphase bzw. über die gesamte Exspirationsphase ermittelt und somit eine verhältnismäßig große Datenmenge verarbeitet werden. Beispielsweise kann als Exspirationsphasenabschnitt ein vorbestimmter Zeitabschnitt identifiziert werden, welcher eine vorbestimmte Zeitspanne nach Beginn der Exspirationsphase anfängt und eine vorbestimmte Zeitspanne lang andauert. In einem solchen Exspirationsphasenabschnitt als einem in der Regel plateauartigen Atemhub-Teilphasenabschnitt mit, verglichen mit den transienten Abschnitten zu Beginn und am Ende einer Atemhub-Teilphase, verhältnismäßig geringen betragsmäßigen Änderungen des O₂-Sensorsignals - aber nicht nur in einem solchen Atemhub-Teilphasenabschnitt - kann ein charakteristischer exspiratorischer O₂-Gehaltswert als Mittelwert einer Mehrzahl von O₂-Gehaltswerten bestimmt werden. Für den Inspirationsphasenabschnitt als dem jeweils anderen Atemhub-Teilphasenabschnitt gilt das Entsprechende.

Jede Atemhub-Teilphase zeigt hinsichtlich der hier diskutierten Gasanteile: Sauerstoff und Kohlenstoffdioxid üblicherweise einen anfänglichen transienten Abschnitt mit raschem Anstieg des Gas-Gehaltswerts am Beatmungsgas. Dem transienten Abschnitt folgt ein Plateauabschnitt mit betragsmäßig geringen Änderungen des Gas-Gehaltswerts, der im Plateauabschnitt um ein gewisses Niveau herum schwankt. Auf den Plateauabschnitt folgt ein endseitiger transienter Abschnitt, in welchem der Gas-Gehaltswert rasch abnimmt.

Der Beginn oder/und das Ende einer Atemhub-Teilphase kann aus den erfassten Beatmungsgasströmen in der Beatmungsleitungsanordnung ermittelt werden. Zur Erfassung von Beatmungsgasströmen weist die Beatmungsvorrichtung in der Beatmungsleitungsanordnung bevorzugt einen Flusssensor auf, etwa einen nach dem Differenzdruckprinzip arbeitenden Flusssensor oder einen Heißdrahtsensor. Alternativ oder zusätzlich kann der Beginn oder das Ende einer Atemhub-Teilphase aus einer Erfassung eines Betriebs der Druckveränderungsanordnung, beispielsweise aus einer Betriebsstellung eines Inspirationsventils und eines Exspirationsventils oder aus einem Betrieb eines Gebläses der Druckveränderungsanordnung, bestimmt werden.

Alternativ kann ein Atemhub-Teilphasenabschnitt zur Bestimmung eines charakteristischen O₂-Gehaltswerts unabhängig von einer vorbestimmten Zeitspanne auf Grundlage eines vorbestimmten Schwellenwerts ermittelt werden, etwa auf Grundlage eines vorbestimmten zeitlichen Abstands zu dem Ereignis eines Erreichens eines Schwellenwertes. Der vorbestimmte zeitliche Abstand kann auch Null sein. So kann ein Beginn oder ein Ende eines Atemhub-Teilphasenabschnitts erkannt werden basierend auf dem Ereignis, dass ein O₂-Sensorsignal einen O₂-Inspirations-Schwellenwert während einer Inspirationsphase überschreitet, was indiziert, dass ein transienter Abschnitt zu Beginn der Inspirationsphase zu Ende geht oder/und ein O₂-Sensorsignal einen O₂-Exspirations-Schwellenwert während einer Exspirationsphase unterschreitet, was indiziert, dass ein Plateauabschnitt der Exspirationsphase mit lediglich geringer betragsmäßiger Änderung der O₂-Sensorsignale zu Ende geht und in einen transienten Abschnitt am Ende der Exspirationsphase übergeht.

Um mittels der Beatmungsvorrichtung die Verstoffwechselung von Sauerstoff in der Patientenlunge erfassen zu können, weist die Beatmungsvorrichtung gemäß einer bevorzugten Weiterbildung zusätzlich eine CO₂-Sensoranordnung auf, welche zur Ermittlung eines einen Kohlenstoffdioxidgehalt des in der Beatmungsleitungsanordnung strömenden Beatmungsgases repräsentierenden CO₂-Gehaltswerts ausgebildet ist, wobei die CO₂-Sensoranordnung CO₂-Sensorsignale, welche Information über den CO₂-Gehaltswert enthalten, an die Auswertevorrichtung ausgibt.

Gemäß einer zweiten Ausführungsform der vorliegenden Erfindung, die alternativ oder bevorzugt zusätzlich zur obigen ersten Ausführungsform realisiert sein kann, kann die Auswertevorrichtung dazu ausgebildet sein, aus den CO₂-Sensorsignalen einen eine zeitliche Änderung des Kohlenstoffdioxidgehalts im exspiratorischen Beatmungsgas repräsentierenden CO₂-Gradientenwert zu ermitteln. Der Änderung des Kohlenstoffdioxidgehalts im exspiratorischen Beatmungsgas steht die Änderung des CO₂-Gehaltswerts in einer Exspirationsphase gleich. Gemäß dieser zweiten Ausführungsform ist die Auswertevorrichtung weiter dazu ausgebildet, als den O₂-Änderungswert einen eine zeitliche Änderung des Sauerstoffgehalts im inspiratorischen Beatmungsgas repräsentierenden O₂-Gradientenwert zu ermitteln. Der Änderung des Sauerstoffgehalts im inspiratorischen Beatmungsgas steht die Änderung des O₂-Gehaltswerts in einer Inspirationsphase gleich. Die oben genannte vorbestimmte Bedingung ist dann, dass ein Verhältnis des CO₂-Gradientenwerts zu dem O₂-Gradientenwert in einer vorbestimmten relativen Beziehung zu einem Änderungsverhältnisgrenzwert steht. Diese Bedingung und ihre Weiterbildungen sind in der vorliegenden Anmeldung als "Gradientenkriterium" bezeichnet. Die Auswertevorrichtung ist daher gemäß der zweiten Ausführungsform dazu ausgebildet, dann auf eine Degradation der O₂-Sensoranordnung zu schließen und ein entsprechendes Signal auszugeben, wenn ein Verhältnis des CO₂-Gradientenwerts zu dem O₂-Gradientenwert in einer vorbestimmten relativen Beziehung zu einem Änderungsverhältnisgrenzwert steht.

Bezüglich des Begriffs der "vorbestimmten relativen Beziehung" gilt das oben bereits Gesagte.

Die in der vorliegenden Anmeldung genannten Grenzwerte, wie insbesondere Unterschiedsgrenzwert und Änderungsgrenzwert können vorab im Labor bestimmt sein.

Das Gradientenkriterium ist in zu Erläuterungszwecken stark vereinfachter Darstellung ein Mittel zur Beurteilung, ob Änderungen des Kohlenstoffdioxidgehalts im exspiratorischen Beatmungsgas und Änderungen des Sauerstoffgehalts im inspiratorischen Beatmungsgas betragsmäßig ausreichend plausibel zusammenpassen: ändert sich der O₂-Gradientenwert, etwa weil die flüssigkeitsbelastete O₂-Sensoranordnung Sauerstoff in geringerem Umfang und daher mit geringerer zeitlicher Änderung als ohne Flüssigkeitsbelastung erfasst, ohne dass sich der CO₂-Gradientenwert ändert, kann die Auswertevorrichtung dann auf eine Degradation der O₂-Sensoranordnung schließen, wenn das Verhältnis des Betrags des CO₂-Gradientenwerts zu dem Betrag des O₂-Gradientenwerts größer ist als der Änderungsverhältnisgrenzwert. Dem steht gleich, wenn das Verhältnis des Betrags des O₂-Gradientenwerts zu dem Betrag des CO₂-Gradientenwerts kleiner ist als ein weiterer Änderungsverhältnisgrenzwert, wobei der weitere Änderungsverhältnisgrenzwert der Kehrwert des zuvor genannten Änderungsverhältnisgrenzwerts ist. Wegen dieses eindeutigen Zusammenhangs wird nachfolgend nur der zuvor genannte Änderungsverhältnisgrenzwert diskutiert.

Wenngleich es in einer weniger bevorzugten Ausführungsform der Erfindung ausreichen kann, nur eines der beiden oben genannten Kriterien aus Unterschiedskriterium und Gradientenkriterium für die Bestimmung einer Beeinträchtigung der O₂-Sensoranordnung anzuwenden, nutzt die erfindungsgemäße Beatmungsvorrichtung mit dem Ziel einer möglichst sicheren Erfassung einer Degradation der O₂-Sensoranordnung bevorzugt beide Kriterien zur Bestimmung einer Beeinträchtigung der O₂-Sensoranordnung, wobei die Erfüllung bereits eines der Beurteilungskriterien aus Unterschiedskriterium und Gradientenkriterium eine Beeinträchtigung der O₂-Sensoranordnung indiziert und zur Ausgabe eines entsprechenden Signals ausreichen kann.

Um eine Degradation der O₂-Sensoranordnung so frühzeitig wie möglich erfassen zu können, führt die Auswertevorrichtung während eines Beatmungsbetriebs einen Prüfvorgang zur Bestimmung einer Beeinträchtigung der O₂-Sensoranordnung bevorzugt iterativ durch, vorzugsweise in einem mit dem Beatmungsrhythmus in funktionalem Zusammenhang stehenden Rhythmus, etwa für jeden n-ten Atemhub, wobei n eine natürliche Zahl ist. Besonders bevorzugt ist n = 1, so dass für jeden Atemhub ein Prüfvorgang durchgeführt wird.

Das von der Auswertevorrichtung auf ein Erfassen einer Degradation der O₂-Sensoranordnung hin ausgegebene Signal kann ein für einen Benutzer in der Außenumgebung der Beatmungsvorrichtung wahrnehmbares Signal sein, wie etwa ein akustisches oder/und optisches oder/und haptisches Signal, sodass der Benutzer möglichst schnell Kenntnis von der erfassten Degradation erhält. Das Signal kann alternativ oder zusätzlich ein Datensignal sein, welches die Auswertevorrichtung an eine andere Vorrichtung hin zur weiteren Signalverarbeitung durch diese ausgibt, etwa an die Steuervorrichtung oder an einen Abschnitt der Steuervorrichtung.

Grundsätzlich kann der CO₂-Gradientenwert in einem beliebigen transienten Abschnitt einer Exspirationsphase bestimmt werden, also zu Beginn einer Exspirationsphase oder gegen Ende der Exspirationsphase. In den transienten Abschnitten ändert sich der CO₂-Gehaltswert mit der Zeit betragsmäßig besonders stark, so dass in einem transienten Abschnitt ein besonders ausgeprägter CO₂-Gradientenwert ermittelbar ist.

Bisherige Versuche haben gezeigt, dass der Kohlenstoffdioxidgehalt im exspiratorischen Beatmungsgas als Funktion der Zeit, bei ansonsten gleich bleibenden Beatmungsparametern, in den jeweiligen Endabschnitten von Exspirationsphasen besser untereinander übereinstimmende Werteverläufe liefert als in den jeweiligen Anfangsabschnitten der Exspirationsphasen. Mit anderen Worten: die fallenden Flanken einer Kohlenstoffdioxidgehaltkurve als Funktion der Zeit in einem vorgegebenen Beatmungsbetrieb stimmen unter konstanten Beatmungsparametern besser überein als die steigenden Flanken derselben Kurve. Aus diesem Grunde ist die Auswertevorrichtung bevorzugt dazu ausgebildet, den CO₂-Gradientenwert im Endabschnitt einer Exspirationsphase bzw. im Wechselabschnitt von einer Exspirationsphase zu einer Inspirationsphase zu bestimmen, also in der fallenden Flanke bzw. den fallenden Flanken einer den Kohlenstoffdioxidgehalt des Beatmungsgases über die Beatmungsdauer hinweg anzeigenden Kohlenstoffdioxidgehaltkurve. Der CO₂-Gradientenwert ist daher bevorzugt ein Wert einer Änderung des Kohlenstoffdioxidgehalts bzw. des ihn repräsentierenden CO₂-Gehaltswerts zu kleineren Werten hin.

Da die Beatmungsvorrichtung eine Exspirationsphase durch Zuführung von inspiratorischem Beatmungsgas zum Patienten beenden kann, kann der Endabschnitt einer Exspirationsphase mit dem Anfangsabschnitt einer Inspirationsphase zeitlich überlappen. Da ein Exspirationsvorgang beginnt, indem in der Patientenlunge unter Druck stehendes Beatmungsgas beginnt, aus der Patientenlunge gegen den betragsmäßig abnehmenden Strom von inspiratorischem Beatmungsgas vom Patienten weg zu strömen, kann auch der Endabschnitt einer Inspirationsphase mit dem Anfangsabschnitt einer Exspirationsphase überlappen.

Der CO₂-Gradientenwert kann von der Auswertevorrichtung auf unterschiedliche Art und Weise ermittelt werden, etwa in der Art eines Steigungsdreiecks durch Subtraktion eines zeitlich früheren CO₂-Gehaltswerts von einem zeitlich späteren CO₂-Gehaltswert und durch Division des so erhaltenen Subtraktionswerts durch den zeitlichen Abstand zwischen den beiden CO₂-Gehaltswerten. Alternativ oder zusätzlich kann der zeitliche Verlauf einer Kohlenstoffdioxidgehaltkurve bzw. einer CO₂-Gehaltswertekurve, gestützt auf zu unterschiedlichen Zeiten erhaltene CO₂-Sensorsignale, wenigstens abschnittsweise durch eine analytische mathematische Funktion angenähert und die Funktion zur Ermittlung des CO₂-Gradientenwerts nach der Zeit differenziert werden. Entsprechendes gilt für die Bestimmung des O₂-Gradientenwerts.

Grundsätzlich kann auch ein über eine Mehrzahl von in einem vorbestimmten Zeitintervall gelegenen CO₂-Gradientenwerten ermittelter Durchschnittswert als der CO₂-Gradientenwert ermittelt werden. Es hat sich jedoch als vorteilhaft erwiesen, bei Anwendung des Gradientenkriteriums den CO₂-Gradientenwert als Bezugswert zur Bestimmung des O₂-Gradientenwerts heranzuziehen. Folglich ist ein CO₂-Gradientenwert bevorzugt, dem mit ausreichender Genauigkeit ein Zeitpunkt seines Auftretens zugeordnet werden kann.

Bevorzugt ist daher die Auswertevorrichtung dazu ausgebildet, einen charakteristischen CO₂-Gradientenwert als einen Bezugswert zu bestimmen. Dies kann ein in einem vorbestimmten Abschnitt der Exspirationsphase auftretender Extremwert eines CO₂-Gradientenwerts sein. Dieser Extremwert kann ein lokaler oder ein absoluter Extremwert in einem transienten Abschnitt einer Exspirationsphase sein. Treten beispielsweise in einem bestimmten Beatmungsbetrieb eine Mehrzahl von lokalen Extremwerten des CO₂-Gradientenwerts in dem transienten Abschnitt auf, kann der k-te auftretende lokale Extremwert als der charakteristische CO₂-Gradientenwert herangezogen werden, wobei k eine vorbestimmte natürliche Zahl ist.

Innerhalb eines vorbestimmten Abschnitts einer Exspirationsphase ist die Ermittlung eines absoluten Extremwerts als der charakteristische CO₂-Gradientenwert wegen der Eindeutigkeit des so ermittelten Bezugswerts bevorzugt. Daher ist gemäß den obigen Ausführungen die Auswertevorrichtung bevorzugt dazu ausgebildet ist, auf Grundlage einer Mehrzahl von CO₂-Sensorsignalen eines Atemhubs, insbesondere auf Grundlage einer Mehrzahl von CO₂-Sensorsignalen einer Exspirationsphase, die betragsmäßig größte zeitliche Änderung des Kohlenstoffdioxidgehalts hin zu kleiner werdenden Werten als den CO₂-Gradientenwert zu ermitteln.

Zur Bestimmung des O₂-Gradientenwerts kann die Auswertevorrichtung dazu ausgebildet sein, den Zeitpunkt des Auftretens des CO₂-Gradientenwerts als Bezugszeitpunkt zu verwenden und auf Grundlage einer Mehrzahl von O₂-Sensorsignalen eines Atemhubs einen Änderungswert des Sauerstoffgehalts als den O₂-Gradientenwert zu verwenden, welcher in einem vorbestimmten zeitlichen Abstand vom CO₂-Gradientenwert auftritt. Der zeitliche Abstand kann ein vorbestimmter zeitlicher Abstand sein oder kann anhand von Beatmungsparametern, insbesondere von der Beatmungsfrequenz, für den jeweiligen Beatmungsbetrieb bestimmt werden.

Grundsätzlich kann auch der O₂-Gradientenwert in einem beliebigen transienten Abschnitt eines Atemhubs, insbesondere in einer Inspirationsphase, ermittelt werden. Hier hat sich herausgestellt, dass eine Degradation der O₂-Sensoranordnung gerade zu Beginn einer Inspirationsphase besonders deutlich zutage tritt, wenn der Sauerstoffgehalt im Beatmungsgas von dem niedrigen Wert einer Exspirationsphase ausgehend ansteigt. Bevorzugt ist daher zur Erzielung besonders sicherer Beurteilungsergebnisse hinsichtlich einer Degradation der O₂-Sensoranordnung vorgesehen, dass der vorbestimmte zeitliche Abstand derart gewählt ist, dass der O₂-Gradientenwert in einem Anfangsabschnitt einer Inspirationsphase oder, wegen der oben genannten Möglichkeit der zeitlichen Überlappung von Inspirationsphase und Exspirationsphase, in einem Abschnitt eines Wechsels von einer Exspirationsphase zu einer Inspirationsphase gelegen ist. In derartigen Abschnitten steigt der Sauerstoffgehalt mit zeitlichem Fortschritt an.

Bevorzugt tritt der CO₂-Gradientenwert zeitlich also vor dem O₂-Gradientenwert auf.

Wie oben bereits dargelegt wurde, ist der CO₂-Gradientenwert bevorzugt in einem Endabschnitt einer Exspirationsphase oder in einem Abschnitt eines Wechsels von einer Exspirationsphase zu einer Inspirationsphase gelegen. Um möglichst aussagekräftige CO₂- und O₂-Änderungswerte zur Beurteilung der Beeinträchtigung der O₂-Sensoranordnung heranzuziehen, ist es vorteilhaft wenn die ermittelten Gradientenwerte von CO₂- und O₂ zeitlich nicht zu weit auseinanderliegen. Für eine herkömmliche Beatmung eines erwachsenen Patienten liegt der vorbestimmte zeitliche Abstand zwischen dem CO₂-Gradientenwert und dem mit diesem in das Verhältnis gesetzte O₂-Gradientenwert in einem Bereich von 25 bis 80 ms, vorzugsweise in einem Bereich von 35 bis 65 ms, besonders bevorzugt in einem Bereich von 45 bis 55 ms. Dann ist der O₂-Gradientenwert in einem Inspirationsvorgang gelegen, welcher unmittelbar auf den Exspirationsvorgang folgt, in dem der CO₂-Gradientenwert liegt.

Da der charakteristische CO₂-Gradientenwert bevorzugt als Bezugswert für die Prüfvorgänge der vorliegenden Erfindung herangezogen wird, kann die Auswertevorrichtung weiter alternativ zu den oben bereits vorgestellten Ausführungsformen dazu ausgebildet sein, die Atemhub-Teilphasenabschnitte: Inspirationsphasenabschnitt oder/und Exspirationsphasenabschnitt, zur Bestimmung des charakteristischen inspiratorischen O₂-Gehaltswerts oder/und des charakteristischen exspiratorischen O₂-Gehaltswerts ausgehend von dem oben genannten charakteristischen CO₂-Gradientenwert zu identifizieren. Gemäß einer bevorzugten Ausführungsform kann die Auswertevorrichtung dazu ausgebildet sein, den Inspirationsphasenabschnitt oder/und den Exspirationsphasenabschnitt in Abhängigkeit von der zeitlichen Bestimmung des Auftretens der betragsmäßig größten zeitlichen Änderung des Kohlenstoffdioxidgehalts bzw. des CO₂-Gehaltswerts hin zu kleiner werdenden Werten zu ermitteln. Dies ist vor allem dann von Vorteil, wenn die Auswertevorrichtung, wie es bevorzugt ist, das Unterschiedskriterium und das Gradientenkriterium zur Ermittlung einer Beeinträchtigung der O₂-Sensoranordnung anwendet, so dass ein betragsmäßiges Maximum der zeitlichen Änderung des Kohlenstoffdioxidgehalts bzw. des CO₂-Gehaltswerts hin zu kleiner werdenden Werten als der charakteristische Gradientenwert ohnehin ermittelt wird und dann vorliegt.

Zur Verringerung der zur Bestimmung einer Beeinträchtigung der O₂-Sensoranordnung beanspruchten Systemressourcen kann bei Anwendung des Gradientenkriteriums, ebenso wie zuvor bei Anwendung des Unterschiedskriteriums, die Auswertevorrichtung dazu ausgebildet sein, in einem Endabschnitt eines Exspirationsvorgangs bzw. in einem Wechselabschnitt von einer Exspirationsphase zu einer Inspirationsphase ein Untersuchungszeitintervall zu identifizieren und nur innerhalb des Untersuchungszeitintervalls nach einem Auftreten der betragsmäßig größten zeitlichen Änderung des Kohlenstoffdioxidgehalts bzw. des CO₂-Gehaltswerts hin zu kleiner werdenden Werten zu suchen. Dies verringert den zur Bestimmung der betragsmäßig größten zeitlichen Änderung des Kohlenstoffdioxidgehalts notwendigen Rechenaufwand erheblich.

Da ein valides, also taugliches Untersuchungszeitintervall in der Regel an den oben genannten Plateauabschnitt anschließt, können die oben genannten Kriterien zur Identifikation der Atemhub-Teilphasenabschnitte in der Anwendung des Unterschiedskriteriums auch zur Identifikation des Untersuchungszeitintervalls bei Anwendung des Gradientenkriteriums herangezogen werden. Folglich kann die Auswertevorrichtung dazu ausgebildet sein, zur Identifikation des Untersuchungszeitintervalls aus einer Mehrzahl an CO₂-Sensorsignalen als ein Auslöseereignis zu bestimmen, wann der CO₂-Gehaltswert einen vorbestimmten CO₂-Auslösegrenzwert unterschreitet, und ausgehend von dem ermittelten Auslösezeitereignis ein Zeitintervall als ein Kandidatenintervall zu definieren. Der CO₂-Auslösegrenzwert kann ein CO₂-Partialdruck oder/und ein CO₂-Partialvolumen sein, welcher bzw. welches so niedrig gewählt ist, dass er bzw. es durch die üblicherweise zu erwartenden Schwankungen der CO₂-Gehaltswerte im Plateauabschnitt einer Exspirationsphase nicht erreicht wird. Beispielsweise kann ein tauglicher CO₂-Auslösegrenzwert, welcher zu keinen oder nahezu keinen fehlerhaften Auslösungen führt, im Bereich von 5 bis 20 mbar CO₂-Partialdruck, bevorzugt im Bereich von 7,5 bis 15 mbar CO₂-Partialdruck, besonders bevorzugt bei 10 mbar CO₂-Partialdruck liegen.

Das so definierte Kandidatenintervall kann mit wenig Rechenleistung und geringer Speicherplatzanforderung sehr schnell anhand wenigstens eines Validierungskriteriums entweder als Untersuchungszeitintervall validiert oder als Kandidatenintervall verworfen werden.

In Bezug auf die Validierung kann die Auswertevorrichtung konkret dazu ausgebildet sein, das Kandidatenintervall dann als das Untersuchungszeitintervall zu validieren und zu verwenden, wenn die Auswertevorrichtung in einem Validierungsvorgang ermittelt, dass in dem Kandidatenintervall ein CO₂-Gehaltswerteniveau, welches während einer Exspirationsphase, die wenigstens den Beginn des Kandidatenintervalls enthält, oder welches zu Beginn des Kandidatenintervalls vorherrscht, um wenigstens einen vorbestimmten Validierungsbetrag oder/und um wenigstens einen vorbestimmten Validierungsanteil abnimmt.

Durch Anwendung wenigstens eines der, vorzugsweise aller der oben genannten, Validierungskriterien kann sichergestellt werden, dass das aus dem Kandidatenintervall durch Validierung hervorgehende Untersuchungszeitintervall tatsächlich einen ausreichend starken betragsmäßigen Abfall der CO₂-Gehaltswerte aufweist, um einen tauglichen, aussagekräftigen charakteristischen CO₂-Gradientenwert in dem Untersuchungszeitintervall bestimmen zu können.

Beispielsweise kann ein vorbestimmter Validierungsbetrag 2,25 Vol.-% Volumenanteil repräsentieren, was bei Standardatmosphäre etwa 23 mbar Partialdruck entspricht. Nimmt beispielsweise innerhalb eines Kandidatenintervalls der CO₂-Gehaltswert wenigstens um einen Betrag ab, welcher 2,25 Vol.-% Volumenanteil oder 23 mbar Partialdruck von Kohlenstoffdioxid im Beatmungsgas repräsentiert, dann kann das Kandidatenintervall als Untersuchungszeitintervall verwendet werden. Anstelle der genannten betragsmäßig vorbestimmten Validierungsbeträge kann ein Validierungsanteil, etwa 30 %, herangezogen werden. Der Validierungsanteil sollte deutlich größer sein als die gewöhnliche Schwankungsbreite des CO₂-Gehaltswerts im Plateauabschnitt einer Exspirationsphase, um eine fehlerhafte Validierung möglichst sicher auszuschließen.

Da nicht damit zu rechnen ist, dass bereits jedes erste Kandidatenintervall während einer Beatmung zu einem validen Untersuchungszeitintervall wird, ist die Auswertevorrichtung bevorzugt dazu ausgebildet ist, den Validierungsvorgang ab dem Auslöseereignis iterativ mit einem jeweils modifizierten Kandidatenintervall auszuführen. Die Modifikation kann beispielsweise darin bestehen, dass das modifizierte Kandidatenintervall bezüglich des unmittelbar vorhergehenden verworfenen Kandidatenintervalls ein vorbestimmtes Zeitinkrement später beginnt. Beispielsweise kann eine Folge von modifizierten Kandidatenintervallen mit einem zeitlichen Abstand eines ganzzahligen Vielfachen von 10 ms vom Auslösezeitpunkt beginnen, wobei das Vielfache beispielsweise der aktuellen Anzahl an Modifikationen des Kandidatenintervalls oder der Anzahl an bereits verworfenen Kandidatenintervallen entspricht. Der Wert von 10 ms ist hierbei nur beispielhaft genannt. Es kann stattdessen auch ein Wert von 5 ms oder 15 ms oder 20 ms verwendet werden.

Der Validierungsbetrag bzw. der Validierungsanteil kann für unterschiedliche Beatmungssituationen unterschiedlich sein, etwa weil sich die Schwankungsbreite des CO₂-Gehaltswerts in den Plateauabschnitten der Exspirationsphasen von Patient zu Patient oder selbst während der Beatmung ein und desselben Patienten abhängig von dessen Zustand ändern kann. Um eine solche Änderung bei der Validierung von Kandidatenintervallen berücksichtigen zu können, ist die Auswertevorrichtung bevorzugt dazu ausgebildet ist, den vorbestimmten Validierungsbetrag oder/und den vorbestimmten Validierungsanteil auf Grundlage zurückliegender CO₂-Sensorsignale zu ermitteln, etwa auf Grundlage eines mittleren CO₂-Gehaltswerteniveaus während einer vorbestimmten Anzahl zurückliegender exspiratorischer Plateauabschnitte.

Die Beatmungsgasquelle der Beatmungsvorrichtung kann ein Beatmungsgasvorrat oder ein Anschluss zur Verbindung mit einem solchen Vorrat sein, etwa ein Anschluss zur Verbindung mit einem in einem Gebäude, wie etwa einer Klinik, installierten Beatmungsgasvorrat. Die Beatmungsgasquelle kann beispielsweise dann, wenn als Beatmungsgas Umgebungsluft angesaugt wird, ein Gebläse sein, mittels welchem Umgebungsluft ansaugbar und in der Beatmungsleitungsanordnung förderbar ist.

Die Druckveränderungsanordnung kann ein Ventil umfassen oder sein, insbesondere ein hinsichtlich seines Durchlassquerschnitts einstellbares Ventil. Zusätzlich oder alternativ kann die Druckveränderungsanordnung ein Gebläse umfassen oder sein, durch welches Beatmungsgas in der Beatmungsleitungsanordnung gefördert werden kann. Allgemein kann die Druckveränderungsanordnung eine Fördervorrichtung mit einer Saugseite und einer Druckseite sein. Ein Gebläse kann sowohl Beatmungsgasquelle als auch Druckveränderungsanordnung sein.

Die vorliegende Erfindung wird nachfolgend anhand der beiliegenden Zeichnungen näher erläutert. Es stellt dar:

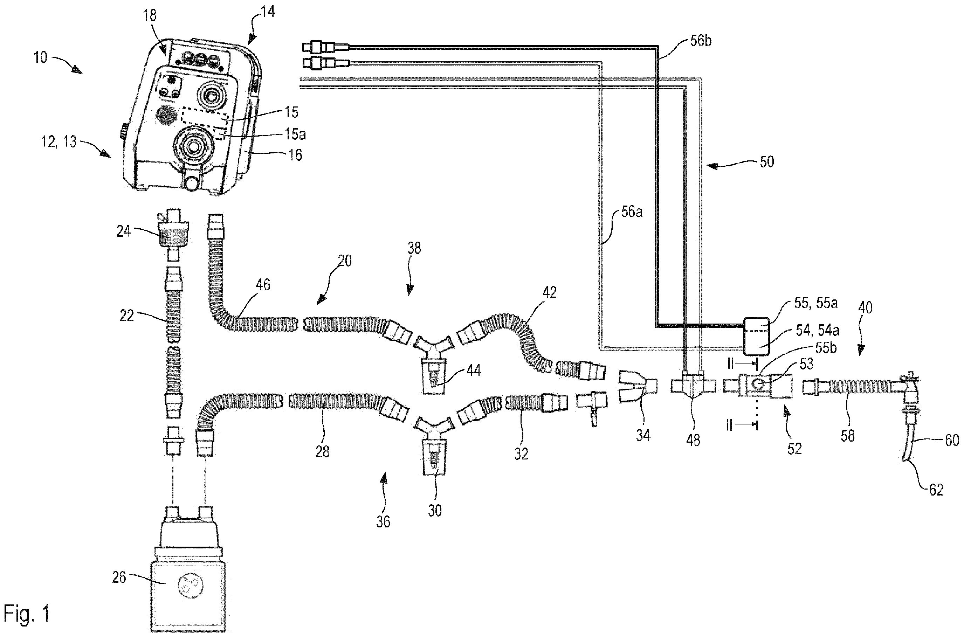

- Fig. 1: eine grobschematische Explosionsdarstellung einer erfindungsgemäßen Beatmungsvorrichtung,

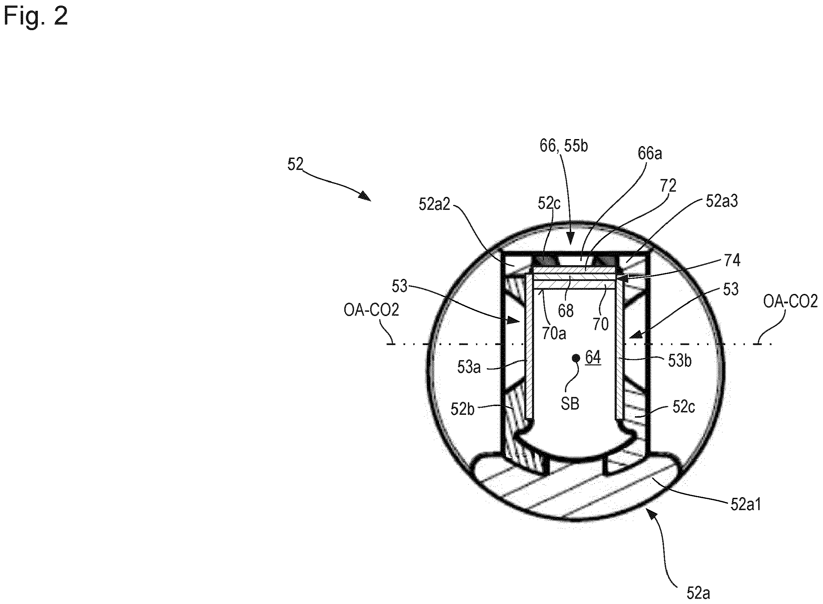

- Fig. 2: eine grobschematische Schnittansicht durch die Messküvette 52 von Figur 1 entlang der zur Strömungsbahn SB der Messküvette 52 orthogonalen Schnittebene II-II von Fig. 1,

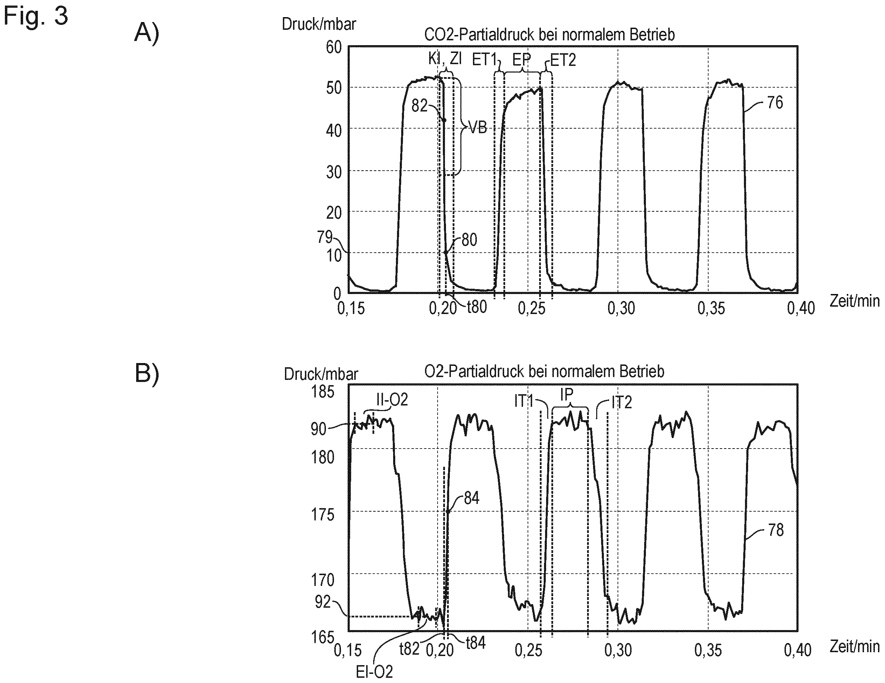

- Fig. 3A: eine grobschematische grafische Darstellung eines zeitlichen Verlaufs eines CO₂-Partialdrucks von in der Messküvette 52 von Figur 1 strömendem Beatmungsgas, wie er während eines Beatmungsbetriebs von der CO₂-Sensoranordnung 54 in Figur 1 erfasst wird, bei normaler, von Feuchtigkeit unbelasteter Messküvette,
- Fig. 3B: eine grobschematische grafische Darstellung eines zeitlichen Verlaufs eines O₂-Partialdrucks von in der Messküvette 52 von Figur 1 strömendem Beatmungsgas, wie er während eines Beatmungsbetriebs von der O₂-Sensoranordnung 55 in Figur 1 erfasst wird, bei normaler, von Feuchtigkeit unbelasteter Messküvette,

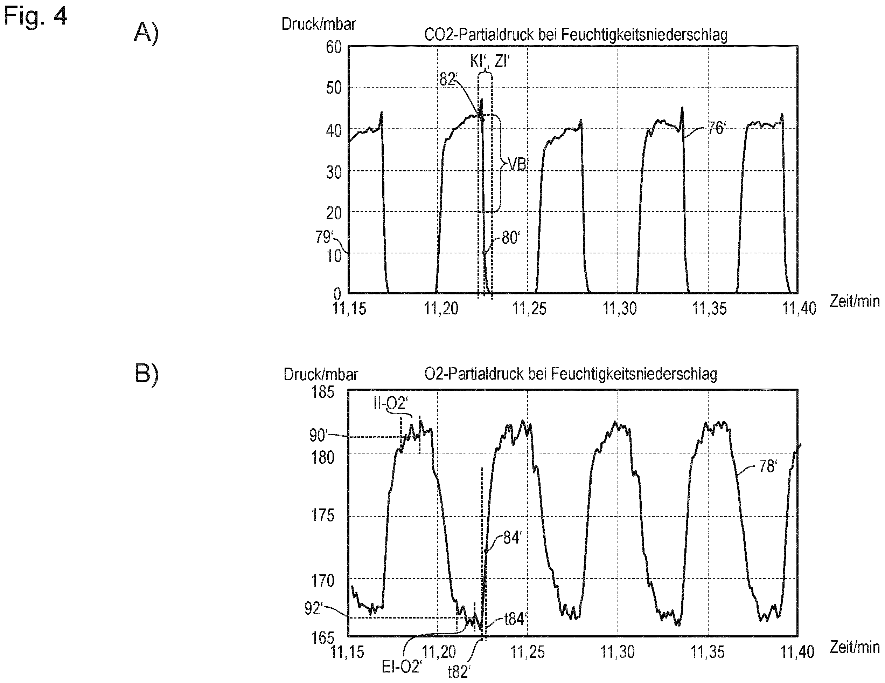

- Fig. 4A: eine grobschematische grafische Darstellung eines zeitlichen Verlaufs eines CO₂-Partialdrucks von in der Messküvette 52 von Figur 1 strömendem Beatmungsgas, wie er während eines Beatmungsbetriebs von der CO₂-Sensoranordnung 54 in Figur 1 erfasst wird, bei mit etwas Feuchtigkeit belasteter Messküvette,
- Fig. 4B: eine grobschematische grafische Darstellung eines zeitlichen Verlaufs eines O₂-Partialdrucks von in der Messküvette 52 von Figur 1 strömendem Beatmungsgas, wie er während eines Beatmungsbetriebs von der O₂-Sensoranordnung 55 in Figur 1 erfasst wird, bei mit etwas Feuchtigkeit belasteter Messküvette,

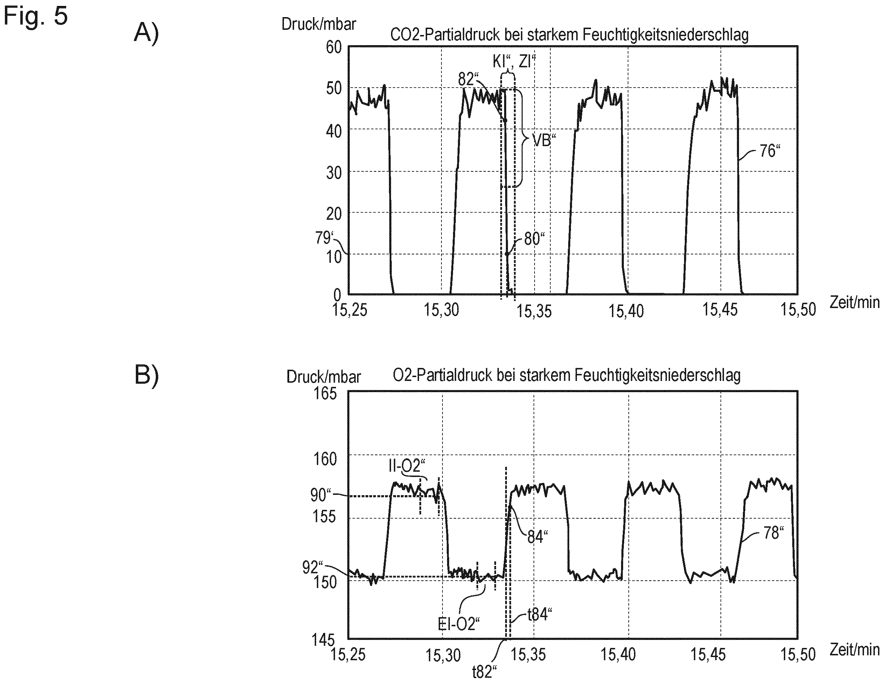

- Fig. 5A: eine grobschematische grafische Darstellung eines zeitlichen Verlaufs eines CO₂-Partialdrucks von in der Messküvette 52 von Figur 1 strömendem Beatmungsgas, wie er während eines Beatmungsbetriebs von der CO₂-Sensoranordnung 54 in Figur 1 erfasst wird, bei stark mit Feuchtigkeit belasteter Messküvette, und
- Fig. 5B: eine grobschematische grafische Darstellung eines zeitlichen Verlaufs eines O₂-Partialdrucks von in der Messküvette 52 von Figur 1 strömendem Beatmungsgas, wie er während eines Beatmungsbetriebs von der O₂-Sensoranordnung 55 in Figur 1 erfasst wird, bei stark mit Feuchtigkeit belasteter Messküvette.

In Figur 1 ist eine erfindungsgemäße Ausführungsform einer Beatmungsvorrichtung allgemein mit 10 bezeichnet. Die Beatmungsvorrichtung 10 umfasst eine Beatmungsgasquelle 12 in Gestalt eines Gebläses sowie eine Steuervorrichtung 14 zur Einstellung von Betriebsparametern der Beatmungsgasquelle 12. Die Beatmungsgasquelle 12 und die Steuervorrichtung 14 sind im selben Gehäuse 16 aufgenommen. In diesem Gehäuse befinden sich auch an sich bekannte Ventile, wie ein Inspirationsventil und ein Exspirationsventil. Diese sind in Figur 1 jedoch nicht eigens dargestellt.

Die Steuervorrichtung 14 umfasst eine Auswertevorrichtung 15 zur Verarbeitung von Daten übertragenden Signalen von weiter unten näher erläuterten Sensoranordnungen 54, 55 und auch 48. Zur Speicherung eines Betriebsprogramms und zur Speicherung von zu verarbeitenden Daten sowie von Zwischen- und Endergebnissen der Datenverarbeitung weist die Auswertevorrichtung 15 einen Datenspeicher 15a auf, welcher von der Auswertevorrichtung 15 gelesen und zumindest abschnittsweise beschrieben werden kann. Die Auswertevorrichtung 15 oder Teile davon kann bzw. können in unmittelbarer Nähe zu wenigstens einer der Sensoranordnungen 54, 55 und auch 48 angeordnet sein. Auch in den Gehäusen von Sensoranordnungen können als intelligente Sensoranordnungen eigene Bausteine, wie Mikroprozessoren und Speichereinheiten, zur Signalverarbeitung der Sensorsignale der jeweiligen Sensoranordnung aufgenommen sein.

Die Steuervorrichtung 14 der Beatmungsvorrichtung 10 weist eine Eingabe/Ausgabevorrichtung 18 auf, welche zahlreiche Schalter, wie Tastschalter und Drehschalter umfasst, um erforderlichenfalls Daten in die Steuervorrichtung 14 eingeben zu können. Das Gebläse der Beatmungsgasquelle 12 kann in seiner Förderleistung durch die Steuervorrichtung 14 verändert werden, um die Menge an Beatmungsgas, das von der Beatmungsgasquelle pro Zeiteinheit gefördert wird, zu verändern. Die Beatmungsgasquelle 12 ist daher im vorliegenden Ausführungsbeispiel auch eine Druckveränderungsvorrichtung 13 der Beatmungsvorrichtung 10.

An die Beatmungsgasquelle 12 ist eine Beatmungsleitungsanordnung 20 angeschlossen, welche im vorliegenden Beispiel fünf flexible Schläuche umfasst. Ein erster inspiratorischer Beatmungsschlauch 22 verläuft von einem zwischen der Beatmungsgasquelle 12 und ihm selbst angeordneten Filter 24 zu einer Konditionierungsvorrichtung 26, wo das von der Beatmungsgasquelle 12 gelieferte Beatmungsgas auf einen vorgegebenen Feuchtegrad befeuchtet und gegebenenfalls mit Aerosol-Medikamenten versehen wird. Der Filter 24 filtert und reinigt die vom Gebläse als der Beatmungsgasquelle 12 gelieferte Umgebungsluft.

Ein zweiter inspiratorischer Beatmungsschlauch 28 führt von der Konditionierungsvorrichtung 26 zu einer inspiratorischen Wasserfalle 30. Ein dritter inspiratorischer Beatmungsschlauch 32 führt von der Wasserfalle 30 zu einem Y-Verbinder 34, welcher die distale Inspirationsleitung 36 und die distale Exspirationsleitung 38 zu einer kombinierten proximalen inspiratorisch-exspiratorischen Beatmungsleitung 40 verbindet.

Vom Y-Verbinder 34 zurück zum Gehäuse 16 verläuft ein erster exspiratorischer Beatmungsschlauch 42 zu einer exspiratorischen Wasserfalle 44 und von dort ein zweiter exspiratorischer Beatmungsschlauch 46 zum Gehäuse 16, wo das exspiratorische Beatmungsgas über ein nicht dargestelltes Exspirationsventil in die Umgebung abgelassen wird.

Auf der proximalen, also patientennahen, kombinierten inspiratorisch-exspiratorischen Seite des Y-Verbinders 34 folgt unmittelbar auf den Y-Verbinder 34 ein Durchflusssensor 48, hier: ein Differenzdruck-Durchflusssensor 48, welcher den inspiratorischen und exspiratorischen Fluss an Beatmungsgas zum Patienten hin und vom Patienten weg erfasst. Eine Leitungsanordnung 50 überträgt den beiderseits eines Strömungshindernisses im Durchflusssensor 48 herrschenden Gasdruck an die Steuervorrichtung 14, die aus den übertragenen Gasdrücken und insbesondere aus der Differenz der Gasdrücke die Menge an pro Zeiteinheit strömendem inspiratorischem und exspiratorischem Beatmungsgas errechnet.

In Richtung vom Y-Verbinder 34 weg folgt zum Patienten hin auf den Durchflusssensor 48 eine Messküvette 52 sowohl zur nicht-dispersiven Infrarot-Erfassung eines CO₂-Anteils im Beatmungsgas als auch zur Erfassung eines O₂-Anteils im Beatmungsgas durch Lumineszenzlöschung. Dabei sind die CO₂- und die O₂-Anteile sowohl im inspiratorischen Beatmungsgas wie auch im exspiratorischen Beatmungsgas von Interesse, da die Änderung des CO₂-Anteils zwischen Inspiration und Exspiration ein Maß für die Stoffwechselfähigkeit der Patientenlunge ist. Zu erkennen ist in Figur 1 eines der seitlichen Fenster 53, durch welche Infrarotlicht in die Messküvette 52 eingestrahlt werden kann bzw. aus dieser ausstrahlen kann, je nach Orientierung eines mit der Messküvette lösbar gekoppelten Mehrkanal-Infrarot-Gassensors 54a als einer CO₂-Sensoranordnung 54.

Auf der Oberseite der CO₂-Sensoranordnung 54, vorzugsweise in einem gemeinsamen Sensorgehäuse, befindet sich ein erster, aktiver Teil 55a einer O₂-Sensoranordnung 55, welche in an sich bekannter Weise dazu ausgebildet ist, einen luminophorhaltigen Observationsbereich 66 (s. Fig. 2) als einen zweiten, passiven Teil 55b der O₂-Sensoranordung 55 an der Oberseite der Messküvette 52 durch eine Strahlenquelle zur Lumineszenz anzuregen und das Lumineszenzverhalten durch einen Lumineszenzsensor hinsichtlich Dauer oder/und Intensität zu beobachten.

Die CO₂-Sensoranordnung 54 und der aktive Teil 55a der O₂-Sensoranordnung 55 sind an die Messküvette 52 derart ankoppelbar, dass der Infrarot-Gassensor 54b die Messküvette 52 mit Infrarotlicht durchleuchten kann und dass der aktive Teil 55a der O₂-Sensoranordnung 55 den Observationsbereich 66 der Messküvette 52 anregen und beobachten kann. Aus der Intensität des Infrarotlichts, genauer aus dessen spektraler Intensität, kann in an sich bekannter Weise auf die Menge bzw. den Anteil von CO₂ in dem die Messküvette 52 durchströmenden Beatmungsgas, also auf dessen CO₂-Gehalt geschlossen werden. CO₂ absorbiert Infrarotlicht einer definierten Wellenlängenbande. Die Intensität des Infrarotlichts in dieser Wellenlänge hängt nach Durchgang durch die Messküvette 52 im Wesentlichen von der Absorption des Infrarotlichts dieser Wellenlänge durch CO₂ ab. Ein Vergleich der Intensität des Infrarotlichts der definierten Wellenlänge mit einer Wellenlänge des Infrarotlichts, die zu keinem Absorptionsspektrum eines zu erwartenden Gasanteils im Beatmungsgas gehört, liefert eine Information über den Anteil von CO₂ am Beatmungsgas. Die CO₂-Sensoranordnung 54 ist daher über eine Datenleitung 56a mit der Steuervorrichtung 14 der Beatmungsvorrichtung 10 verbunden und überträgt über die Datenleitung 56a die beschriebenen Intensitätsinformationen an die Steuervorrichtung 14 oder/und an die Auswertevorrichtung 15. Als "Intensitätsinformation" gilt jede mit der Intensität der Infrarotstrahlung in eindeutigem Zusammenhang stehende Information, wie etwa ein aus einer Infrarot-Strahlungsintensität ermittelter CO₂-Gehaltswert. Im Falle der zuvor genannten "intelligenten" Sensoranordnungen kann eine erste Auswertung des Infrarotsignals bereits, wie beschrieben, in der Auswertevorrichtung oder Teil-Auswertevorrichtung in unmittelbarer Nähe zur Sensoranordnung erfolgen.

Der aktive Teil 55a der O₂-Sensoranordnung 55 regt den luminophorhaltigen Observationsbereich 66 der Messküvette 52 zur Lumineszenz an, wobei die Lumineszenz in Abhängigkeit von der Menge an Sauerstoff abgelöscht wird, die den angeregten Luminophor erreicht. Aus der Beobachtung der Dauer und der Intensität der Lumineszenz kann daher auf die Menge oder den Anteil an O₂ am Beatmungsgas, also auf dessen O₂-Gehalt geschlossen werden. Der aktive Teil 55a der O₂-Sensoranordnung 55 ist daher über eine Datenleitung 56b mit der Steuervorrichtung 14 und der Auswertevorrichtung 15 verbunden und überträgt über die Datenleitung 56b aus der Beobachtung des Lumineszenzverhaltens des Observationsbereichs 66 gewonnene Informationen an die Steuervorrichtung 14 und die Auswertevorrichtung 15.

Auf die Messküvette 52 folgt in Richtung zum Patienten hin ein weiteres Schlauchstück 58, an welchen ein Endotrachealtubus 60 als Beatmungsschnittstelle zum Patienten angeordnet ist. Eine proximale Öffnung 62 des Endotrachealtubus 60 ist sowohl Beatmungsgas-Auslassöffnung, durch welche inspiratorisches Beatmungsgas durch den Endotrachealtubus 60 in den Patienten eingeleitet wird, als auch Beatmungsgas-Einlassöffnung, durch welche exspiratorisches Beatmungsgas aus dem Patienten zurück in den Endotrachealtubus 60 geleitet wird.

Am Beispiel von Figur 2 wird nachfolgend die der vorliegenden Anmeldung zugrunde liegende Problematik geschildert. Figur 2 zeigt einen Schnitt entlang der in Figur 1 gezeigten Schnittebene II-II durch die Messküvette 52 von Figur 1, wobei die Schnittebene II-II orthogonal zur Zeichenebene von Figur 1 und damit auch orthogonal zur Strömungsbahn SB orientiert ist, längs welcher ein Messraum 64 im Beatmungsbetrieb bidirektional von Beatmungsgas durchströmt wird.

Der Messraum 64 ist um die Strömungsbahn SB umlaufend von der Messküvette 52 umgeben, welche hierzu ein bevorzugt einstückig spritzgegossenes Küvettengerüst 52a mit einem Bodenabschnitt 52a1 und zwei zueinander und zur Strömungsbahn SB parallelen Rahmenstreben 52a2 und 52a3 aufweist. Seitlich sind zwischen dem Bodenabschnitt 52a1 und jeder Rahmenstrebe 52a2 und 52a3 je eine Seitenwand 52b bzw. 52c eingesetzt. An der dem Bodenabschnitt 52a1 gegenüberliegenden Seite ist zwischen den Rahmenstreben 52a2 und 52a3 eine Deckenwand 52c eingesetzt.

Die Schnittebene II-II verläuft durch die Fenster 53, welche zum Messraum 64 hin jeweils durch eine für Infrarotlicht transparente Scheibe 53a und 53b bedeckt sind. Die Scheiben 53a und 53b können aus einer Folie oder aus einem formstabilen steifen Material gebildet sein. Die optische Achse der O₂-Sensoranordnung 54 liegt in der Schnittebene II-II und ist durch das Bezugszeichen OA-CO₂ bezeichnet.

Die Oberseite der Messküvette 52 weist den zuvor bereits erwähnten Observationsbereich 66 auf, an dem von außen betrachtet zunächst die Ausnehmung 66a auffällt, die die Deckenwand 52c durchsetzt. Durch die Ausnehmung 66a wird der Luminophor einer Luminophorschicht 68 von der nicht dargestellten Strahlenquelle zur Lumineszenz angeregt. Durch die Ausnehmung 66a wird außerdem das Lumineszenzverhalten der Luminophorschicht 68 nach deren Anregung von einem entsprechend ausgestalteten Lumineszenzsensor erfasst.

Die Luminophorschicht 68 ist von einer sauerstoffdurchlässigen Membran 70 getragen, und zwar nur auf der vom Messraum 64 weg weisenden Seite der Membran 70. Eine solche sauerstoffdurchlässige Membran 70 kann beispielsweise aus Polyvinylidenfluorid, kurz PVDF, gebildet sein. Zur Ausnehmung 66a hin ist die Luminophorschicht 68 durch eine sauerstoffundurchlässige Deckschicht 72, beispielsweise aus biaxial orientiertem Polypropylen, gebildet.

Grundsätzlich funktioniert diese kombinierte Anordnung einer CO₂-Sensoranordnung 54 und einer O₂-Sensoranordnung 55 einschließlich des dem passiven Teil 55b der O₂-Sensoranordnung 55 zugehörigen Schichtkörpers 74 aus der Membran 70, der Luminophorschicht 68 und der Deckschicht 72 gut. Es kann jedoch sein, dass sich von dem im Messraum 64 strömenden Beatmungsgas mitgeführte Feuchtigkeit oder/und mitgeführte Körperflüssigkeiten, wie Speichel oder Schleim, an der zum Messraum 64 hinweisenden Seite 70a der Membran 70 niederschlagen und dort einen die Membran 70 teilweise oder vollständig abdeckenden Flüssigkeitsfilm bilden. Aufgrund der Porosität der Membran 70 kann sich auf der Seite 70a niederschlagende Flüssigkeit auch in die Membran 70 hinein migrieren und so die Sauerstoffdurchlässigkeit der Membran 70 verändern.

Wenn ein solcher Niederschlag erfolgt, erreicht Beatmungsgas und vom Beatmungsgas mitgeführter Sauerstoff die Luminophorschicht 68 nur noch in vermindertem Umfang, sodass die durch den Sauerstoff verursachte Lumineszenzlöschung den tatsächlichen Sauerstoffgehalt des Beatmungsgases nicht mehr korrekt wiedergibt.

Da die Erfassung des Kohlenstoffdioxids im Beatmungsgas auf Strahlungsabsorption und nicht auf Molekülkontakt beruht, ist das Ergebnis der Kohlendioxiderfassung durch die CO₂-Sensoranordnung 54 von sich an den Scheiben 53a und 53b niederschlagender Flüssigkeit weitestgehend unbeeinflusst bzw. kann in gewissen Grenzen durch Verwendung einer Referenzstrahlung mit einer weder vom Beatmungsgas noch von der niedergeschlagenen Feuchtigkeit absorbierten Wellenlänge kompensiert werden.

Anhand der Figuren 3A bis 4B wird nachfolgend die Arbeitsweise der Beatmungsvorrichtung 10 zur Ermittlung einer Beeinträchtigung der O₂-Sensoranordnung 55, insbesondere der Luminophorschicht 68, erläutert.

Die Datenverarbeitungsoperationen zur Bestimmung einer Beeinträchtigung der O₂-Sensoranordnung 55 finden während der laufenden Beatmung eines Patienten statt, werden jedoch bevorzugt an im Datenspeicher 15a hinterlegten CO₂-Sensorsignalen und O₂-Sensorsignalen bzw. aus diesen Sensorsignalen ermittelten Gehaltswerten durchgeführt, sodass innerhalb einer Mehrzahl von in einem Zeitintervall zur Verfügung stehenden Datenwerten zeitlich nach vorne und zurück gesprungen werden kann. Da der endliche Datenspeicher 15a während der Bestimmung einer Beeinträchtigung der O₂-Sensoranordnung 55 mit aktuellen Sensorsignalen oder daraus gebildeten Gehaltswerten beschrieben wird, ist eine ressourcenschonende Bestimmung einer Beeinträchtigung mit möglichst geringer Inanspruchnahme von Rechenleistung und Speicherplatz gewünscht, um die Bestimmung anhand von verfügbaren Daten durchführen zu können, bevor diese von aktuelleren Daten überschrieben werden. Beim Überschreiben von Daten werden jeweils die ältesten gespeicherten Daten durch die zu speichernden aktuellen Daten überschrieben.

Figur 3A zeigt grobschematisch in Schritten mit einer Schrittweite von 0,05 Minuten einen unter normalen, insbesondere von Flüssigkeit unbelasteten Betriebsbedingungen, durch die CO₂-Sensoranordnung 54 ermittelten zeitlichen Verlauf 76 des CO₂-Partialdrucks als einen CO₂-Gehaltswert. Da als Beatmungsgas zugeführte Umgebungsluft nahezu kein CO₂ enthält, sind die Exspirationsvorgänge von den einen CO₂-Partialdruck von unter 5 mbar aufweisenden Inspirationsvorgängen gut zu unterscheiden.

Figur 3B zeigt grobschematisch in derselben Zeitskala wie Figur 3A einen während der Erfassung des CO₂-Partialdrucks durch die O₂-Sensoranordnung 55 erfassten zeitlichen Verlauf 78 eines O₂-Partialdrucks im Beatmungsgas, also in Umgebungsluft, als einen O₂-Gehaltswert. Da während eines Atemhubs nur ein Teil des in der Umgebungsluft vorhandenen Sauerstoffs durch den Stoffwechsel des Patienten in CO₂ umgesetzt wird, enthält auch das exspiratorische Beatmungsgas nach wie vor Sauerstoff, jedoch mit niedrigerem Gehalt. Der zeitliche Verlauf 78 des O₂-Partialdrucks verhält sich daher qualitativ invers zum zeitlichen Verlauf 76 des CO₂-Partialdrucks, insofern als der O₂-Partialdruck während eines Inspirationsvorgangs betragsmäßig höhere Werte annimmt als während eines Exspirationsvorgangs.

Da ein Inspirationsvorgang beginnt, indem die Beatmungsgasquelle 12 beginnt, dem Patienten inspiratorisches Beatmungsgas gegen den betragsmäßig abnehmenden Strom von exspiratorischem Beatmungsgas zuzuführen und da ein Exspirationsvorgang beginnt, indem unter Druck stehendes Beatmungsgas anfängt, aus der Patientenlunge gegen den betragsmäßig abnehmenden Strom von inspiratorischem Beatmungsgas vom Patienten weg zu strömen, können sich Inspirations- und Exspirationsphasen zeitlich überlappen.

Anhand des in Figur 3A zweiten Peaks des zeitlichen Verlaufs des CO₂-Partialdrucks werden nachfolgend die Abschnitte erläutert, die ein solcher zeitlicher Verlauf während einer Exspiration aufweist.

Im Wechsel von einer Inspiration zu einer Exspiration ändert sich zwangsweise die Strömungsrichtung des Beatmungsgases in der Beatmungsleitungsanordnung 20 und damit in der Messküvette 52, sodass zu Beginn einer Exspiration ausgehend von einer Strömungsgeschwindigkeit von etwa 0 m/s die Strömungsgeschwindigkeit des exspiratorischen Beatmungsgases und damit der Volumen- und Massenstrom an exspiratorischem Beatmungsgas ansteigt, wodurch auch die vom exspiratorischen Beatmungsgas pro Zeiteinheit im Messraum 64 der Messküvette 52 mitgeführte Menge an Kohlenstoffdioxid ansteigt. Dieser Anstieg an Kohlenstoffdioxidgehalt bildet einen ersten transienten exspiratorischen Abschnitt ET1.

An diesen ersten transienten exspiratorischen Abschnitt ET1 schließt sich ein exspiratorischer Plateauabschnitt EP an, in welchem die Änderung des CO₂-Partialdrucks als dem beispielhaft gewählten CO₂-Gehaltswert erheblich geringer ist als im ersten transienten exspiratorischen Abschnitt ET1. An den exspiratorischen Plateauabschnitt EP schließt sich gegen Ende des Exspirationsvorgangs ein zweiter transienter exspiratorischer Abschnitt ET2 an, in welchem die Strömungsgeschwindigkeit und damit der Massen- und Volumenstrom an exspiratorischem Beatmungsgas abnimmt, insbesondere bis auf 0 m/s abnimmt. Mit dem abnehmenden Strom an exspiratorischem Beatmungsgas nimmt unvermeidlich auch die pro Zeiteinheit an der CO₂-Sensoranordnung 54 vorbeiströmende Menge an Kohlenstoffdioxid ab.

Ebenso weisen die Inspirationsvorgänge zu Beginn und am Ende transiente inspiratorische Abschnitte IT1 und IT2 sowie zwischen den transienten Abschnitten einen inspiratorischen Plateauabschnitt IP auf. Diese Abschnitte sind am dritten Peak in Figur 3B dargestellt. Die Zeitanteile einer Inspirationsphase, die auf die einzelnen Abschnitte IT1, IP und IT2 entfallen, können von den Zeitanteilen der jeweiligen Abschnitte ET1, EP und ET2 einer Exspirationsphase verschieden sein und sind in der Regel wegen der unterschiedlichen strömungsmechanischen Vorgänge und ihrer Ursachen verschieden.

Da die Beatmungsvorrichtung 10 bevorzugt sowohl das Gradientenkriterium als auch das Unterschiedskriterium anwendet, beginnt die Beatmungsvorrichtung 10 mit der Bestimmung eines charakteristischen CO₂-Gradientenwerts, da dieser bevorzugt als Bezugspunkt für weitere Bestimmungen von Werten dient. Es sei jedoch darauf hingewiesen, dass, wie in der Beschreibungseinleitung erläutert, die nachfolgend beschriebenen weiteren Werte auch ohne vorherige Bestimmung eines charakteristischen CO₂-Gradientenwerts bestimmbar sind.

Als aussagekräftigster charakteristischer CO₂-Gradientenwert hat sich in bisherigen Versuchen der Wert betragsmäßig größtmöglicher Steigung des zeitlichen Verlaufs des CO₂-Partialdrucks (oder allgemein eines CO₂-Gehaltswerts) in der fallenden Flanke einer Exspirationsphase erwiesen. Zunächst bestimmt die Auswertevorrichtung 15 möglichst effektiv und mit wenig Rechenaufwand ein Kandidatenintervall Kl, in welchem die fallende Flanke des CO₂-Partialdrucks in einer Exspirationsphase liegen könnte. Dies kann beispielsweise für den zweiten Peak in Figur 3A der dort angezeigte zweite transiente Abschnitt ET2 sein.

Eine von mehreren möglichen Formen der Bestimmung eines Kandidatenintervalls Kl, die sich in bisherigen Versuchen bewährt hat, umfasst zunächst die Bestimmung desjenigen Zeitpunkts, zu welchem der CO₂-Gehaltswert auf ein vorbestimmtes, sicher nicht mehr zum exspiratorischen Plateauabschnitt EP gehörendes Niveau abgefallen ist, beispielsweise an welchem der CO₂-Partialdruck einen CO₂-Auslöse-grenzwert 79 von 20 mbar oder auch von 10 mbar unterschritten hat. In Figur 3A ist der CO₂-Auslösegrenzwert 79 von 10 mbar verwendet, welcher im ersten Exspirationsvorgang am Punkt 80 unterschritten wird. Dieser liegt bei etwa 0,204 Minuten.

Ausgehend von dem so ermittelten Auslöseereignis eines Unterschreitens des vorgegebenen CO₂-Auslösegrenzwerts 79 wird von dem Startzeitpunkt t80, also im Beispiel 0,204 Minuten, das Kandidatenintervall KI definiert, welches eine vorbestimmte erste Zeitspanne vor dem Auslösezeitpunkt t80 beginnt und eine vorbestimmte zweite Zeitspanne nach dem Auslösezeitpunkt t80 endet. Die vorbestimmten ersten und zweiten Zeitspannen sind dabei ausgehend von bisherigen Erfahrungen im Beatmungsbetrieb derart gewählt, dass die fallende Flanke in wenigstens 85 %, vorzugsweise in wenigstens 95 % der Exspirationsphasen im Kandidatenintervall KI liegt.

Anschließend überprüft die Auswertevorrichtung 15, ob das Kandidatenintervall KI ein valides Untersuchungszeitintervall ZI ist, in dem es prüft, ob der CO₂-Gehaltswert ausgehend von dem zu Beginn des Kandidatenintervalls KI herrschenden CO₂-Gehaltswert innerhalb des Kandidatenintervalls KI um wenigstens einen vorbestimmten Validierungsbetrag VB von 23 mbar abfällt. Da dies im dargestellten Kandidatenintervall KI der Fall ist, ist das Kandidatenintervall KI als Untersuchungszeitintervall ZI validiert. Es wird fortan als Untersuchungszeitintervall ZI verwendet. Wie in der Beschreibungseinleitung erläutert, können zusätzlich oder alternativ andere Validierungskriterien zur Validierung des Kandidatenintervalls KI herangezogen werden.

Innerhalb des so definierten Untersuchungszeitintervalls ZI bestimmt die Auswertevorrichtung 15, beispielsweise durch iterative Differenzenbildung zwischen zwei in einem Abstand von Δt gelegenen CO₂-Partialdruckwerten und durch Division der so gebildeten Differenz durch den zeitlichen Abstand Δt eine Folge von CO₂-Partialdruck-Gradientenwerten und wählt daraus den betragsmäßig größten aus. Dieser liegt im dargestellten Beispiel bei Punkt 82.

Anschließend wird der Zeitpunkt t82, zu welchem der betragsmäßig größte CO₂-Partialdruck-Gradientenwert auftritt, als Bezugszeitpunkt verwendet. Durch Addition einer vorbestimmten positiven Zeitspanne zu dem Bezugszeitpunkt t82 wird der später gelegene Zeitpunkt t84 erhalten, der in der steigenden Flanke des zweiten Peaks des Graphen 78 liegt.

An dem so erhaltenen Zeitpunkt t84 wird der Betrag der Steigung des Graphen 78 als der O₂-Gradientenwert ermittelt. Es handelt sich dabei um die Steigung des Graphen 78 im Punkt 84. Es muss sich zur Anwendung des Gradientenkriteriums bei dem Punkt 84 um keinen ausgezeichneten Punkt auf dem Graphen 78 handeln. Es genügt, dass dieser Punkt 84 die vorbestimmte Zeitspanne vom Bezugszeitpunkt t82 entfernt in der steigenden Flanke des zeitlichen Verlaufs der O₂-Gehaltswerte gelegen ist. Die vorbestimmte Zeitspanne ist derart gewählt, dass sie in wenigstens 85 %, vorzugsweise wenigstens 95 %, besonders bevorzugt in 100 % der Inspirationsvorgänge in der steigenden Flanke des zeitlichen Verlaufs des O₂-Gehaltswerts gelegen ist, was bereits dadurch möglich ist, dass aufgrund der mechanisierten Beatmung des Patienten Inspirationsvorgänge vorhersagbar unmittelbar auf Exspirationsvorgänge folgen.

Die Auswertevorrichtung 15 bildet dann einen Quotienten aus den Beträgen der beiden erhaltenen Gradientenwerte: CO₂-Gradientenwert und O₂-Gradientenwert, als ein Änderungsverhältnis der Beträge der Gradientenwerte und vergleicht den Wert des so bestimmten Änderungsverhältnisses mit einem im Datenspeicher 15a hinterlegten vorbestimmten Änderungsverhältnisgrenzwert. Dann, wenn der Quotient der Beträge aus CO₂-Gradientenwert im Zähler und O₂-Gradientenwert im Nenner größer als der vorab im Labor bestimmte Änderungsverhältnisgrenzwert ist, welcher beispielsweise 16,6 betragen kann, gibt die Auswertevorrichtung 15 ein Signal aus, welches anzeigt, dass die O₂-Sensoranordnung 55 und mit ihr die erhaltenen O₂-Sensorsignale degradiert sind. Dies ist vorliegend in Figur 3A und 3B nicht der Fall.

Zusätzlich zum zuvor beschriebenen Gradientenkriterium wendet die Auswertevorrichtung 15 das oben in der Beschreibungseinleitung beschriebene Unterschiedskriterium an. Hierzu wird in Fig. 3B die Differenz zwischen einem charakteristischen inspiratorischen O₂-Gehaltswert, also hier einem O₂-Partialdruck, und einem charakteristischen exspiratorischen O₂-Gehaltswert (Partialdruck) gebildet. Dazu können beispielsweise ausgehend vom bereits bestimmten Bezugszeitpunkt t82 ein bezüglich seines Anfangs und seines Endes relativ zum Bezugszeitpunkt t82 in vorbestimmtem zeitlichen Abstand gelegenes inspiratorisches Zeitintervall II-O₂ und ein ebenso bezüglich seines Anfangs und seines Endes relativ zum Bezugszeitpunkt t82 in vorbestimmtem zeitlichen Abstand gelegenes exspiratorisches Zeitintervall EI-O₂ bestimmt werden. Aus den innerhalb der Intervalle II-O₂ und EI-O₂ gelegenen O₂-Gehaltswerten kann jeweils ein mittlerer O₂-Gehaltswert als der charakteristische inspiratorische O₂-Gehaltswert und der charakteristische exspiratorische O₂-Gehaltswert ermittelt werden. Bevorzugt wird der charakteristische inspiratorische O₂-Gehaltswert in dem Inspirationsvorgang ermittelt, welcher jenem Exspirationsvorgang unmittelbar vorausgeht, in dem der charakteristische CO₂-Gradientenwert ermittelt wird.

In dem in Fig. 3B dargestellten Beispiel ergibt sich für den charakteristischen inspiratorischen O₂-Gehaltswert in dem vorbestimmten inspiratorischen Zeitintervall II-O₂ ein durch Bezugszeichen 90 gekennzeichneter gemittelter inspiratorischer O₂-Gehaltswert von etwa 182 mbar. Für den charakteristischen exspiratorischen O₂-Gehaltswert in dem vorbestimmten exspiratorischen Zeitintervall EI-O₂ ergibt sich ein durch Bezugszeichen 92 gekennzeichneter gemittelter exspiratorischer O₂-Gehaltswert von etwa 166,3 mbar. Der O₂-Unterschiedswert beträgt somit etwa 15,7 mbar. Der atmosphärenbezogene O₂-Unterschiedswert beträgt bei der im Beispiel vorhandenen Atmosphäre mit einem Umgebungsdruck von 1013 mbar etwa 0,015. Diesen atmosphärenbezogenen O₂-Unterschiedswert vergleicht die Auswertevorrichtung 15 mit einem O₂-Unterschiedsgrenzwert, welcher vorab im Labor bestimmt wurde. Dieser kann beispielsweise 0,01 betragen. Unterschreitet der atmosphärenbezogene O₂-Unterschiedswert den O₂-Unterschiedsgrenzwert, ist für den atmosphärenbezogenen O₂-Unterschiedswert das Unterschiedskriterium für eine Signalausgabe erfüllt. Dies ist vorliegend nicht der Fall.

Die Auswertevorrichtung 15 beurteilt die O₂-Sensoranordnung 55 folglich als nicht beeinträchtigt und gibt daher kein Signal aus, welches eine Beeinträchtigung der O₂-Sensoranordnung 55 anzeigt.

In den Figuren 4A und 4B ist ein zeitlicher Verlauf des CO₂-Partialdrucks und des O₂-Partialdrucks während einer Beatmung desselben Patienten mit denselben Beatmungsparametern gezeigt. Allerdings hat sich zwischenzeitlich ein wenig Flüssigkeit an den den Messraum 64 begrenzenden Wänden niedergeschlagen, somit auch an den Fenstern 53 sowie an der Membran 70 der O₂-Sensoranordnung 55.

Die Auswertevorrichtung 15 führt an den Sensorsignalen und den daraus ermittelten Gehaltswerten die gleichen Operationen durch, wie sie zuvor im Zusammenhang mit den Figuren 3A und 3B erläutert wurden. Gleiche und bedeutungsgleiche Werte und Bereiche wie in den Figuren 3A und 3B sind in den Figuren 4A und 4B mit gleichen Bezugszeichen versehen, denen lediglich zur Unterscheidung des feuchten Zustands der O₂-Sensoranordnung 55 vom zuvor diskutierten trockenen Zustand ein Apostroph beigegeben ist. Die zuvor anhand der Figuren 3A und 3B beschriebene Vorgehensweise zur Bestimmung einer Beeinträchtigung der O₂-Sensoranordnung 55 gilt somit auch für die Daten und Datenverläufe der Figuren 4A und 4B.

Wichtig ist hier, dass die fallende Flanke des zeitlichen Verlaufs 76' der CO₂-Partialdrücke in der flüssigkeitsbelasteten Messküvette 52 eine etwa identische Steigung aufweist wie zuvor in der trockenen Messküvette 52. Der am Punkt 82' ermittelte charakteristische CO₂-Gradientenwert zum Zeitpunkt t82' als der betragsmäßig größte "feuchte" CO₂-Gradientenwert im Untersuchungszeitintervall ZI' ist betragsmäßig etwa gleich groß wie der zuvor am Punkt 82 ermittelte "trockene" CO₂-Gradientenwert.

Dagegen verläuft die steigende Flanke der O₂-Gradientenwerte flacher, so dass der im wie zuvor abhängig vom Bezugszeitpunkt t82' bestimmten Zeitpunkt t84' ermittelte "feuchte" O₂-Gradientenwert einen betragsmäßig geringeren Wert aufweist.

Das mit den in den Figuren 4A und 4B gezeigten Daten ermittelte Änderungsverhältnis als Quotient der Beträge aus CO₂-Gradientenwert im Zähler und O₂-Gradientenwert im Nenner wird betragsmäßig größer und übersteigt nun den vorbestimmten Änderungsverhältnisgrenzwert von 16,6. Die Auswertevorrichtung 15 schließt daher auf eine Degradation der O₂-Sensoranordnung 55 und gibt ein entsprechendes Signal aus.

Alternativ zu einer sofortigen Ausgabe des Signals, kann die Auswertevorrichtung das Signal beispielsweise auch erst dann ausgeben, wenn innerhalb von fünf aufeinander folgenden Atemhüben für jeden Atemhub wenigstens ein Kriterium aus Gradientenkriterium und Unterschiedskriterium erfüllt ist.

In dem in Fig. 4B dargestellten Beispiel ergibt sich für den charakteristischen inspiratorischen O₂-Gehaltswert bei dem vorbestimmten inspiratorischen Zeitintervall II-O₂ ein wie oben beschrieben bestimmter, durch Bezugszeichen 90' gekennzeichneter gemittelter inspiratorischer O₂-Gehaltswert von etwa 181,5 mbar. Als der wie oben beschrieben bestimmte charakteristische exspiratorische O₂-Gehaltswert 92' ergibt sich ein gemittelter exspiratorischer O₂-Gehaltswert von etwa 166,7 mbar. Der O₂-Unterschiedswert beträgt somit etwa 14,8 mbar. Der atmosphärenbezogene O₂-Unterschiedswert beträgt bei der im Beispiel vorhandenen Atmosphäre mit einem Umgebungsdruck von 1013 mbar etwa 0,014. Diesen atmosphärenbezogenen O₂-Unterschiedswert vergleicht die Auswertevorrichtung mit dem oben genannten O₂-Unterschiedsgrenzwert von 0,01.

Das Gradientenkriterium ist erfüllt, das Unterschiedskriterium jedoch nicht. Dies kann an der im Beispiel der Figuren 4A und 4B verhältnismäßig geringen niedergeschlagenen Feuchtigkeitsmenge liegen. Zur Bestimmung einer Beeinträchtigung der O₂-Sensoranordnung 55 reicht jedoch bereits die Erfüllung eines der aufgezeigten Kriterien aus.

Die Figuren 5A und 5B zeigen analog zu den Figuren 4A und 4B CO₂- bzw. O₂-Gehaltswerte als Funktion der Zeit bei stärker mit Flüssigkeit verschmutzter O₂-Sensoranordnung 55 als dies im Beispiel der Figuren 4A und 4B der Fall ist. Die Diagramme der Figuren 5A und 5B beruhen auf einer Erfassung von Beatmungsgas in einer Messküvette 52, deren innere Oberfläche mit einer Schleimschicht bedeckt ist. Dies gilt auch für die zum Messraum 64 hinweisende Oberfläche 70a der Membran 70. Gleiche und bedeutungsgleiche Werte und Bereiche wie in den Figuren 3A bis 4B sind mit gleichen Bezugszeichen versehen, jedoch gekennzeichnet durch einen doppelten Apostroph. Mit zunehmender Apostrophenanzahl steigt somit die Flüssigkeitsbelastung der O₂-Sensoranordnung 55.

Wieder werden an den CO₂- und O₂-Gehaltswerten die anhand der Figuren 3A und 3B erläuterten Datenoperationen ausgeführt, um zu beurteilen, ob die O₂-Sensoranordnung 55 beeinträchtigt ist oder nicht.

Anhand des Beispiels der Figuren 5A und 5B soll vor allem das Unterschiedskriterium an einem Beispiel erläutert werden. Wie Figur 5B zeigt, ist durch die stärkere Flüssigkeitsbelastung als im Fall von Figur 4B das Niveau des O₂-Partialdrucks im inspiratorischen Plateauabschnitt von etwa zwischen 181,5 und 182 mbar auf etwa 167 mbar gefallen. Ebenso ist das Niveau des O₂-Partialdrucks im exspiratorischen Plateauabschnitt von etwa 167 mbar auf etwa 151 mbar gefallen.

Das Verhältnis von inspiratorischem zu exspiratorischen mittleren O₂-Partialdruck in den Plateauabschnitten der O₂-Partialdruckkurven 78' und 78" der flüssigkeitsbelasteten O₂-Sensoranordnung 55 liegt in beiden Fällen etwa bei 1,1. Dies gilt ebenso für die 02 Partialdruckkurve 78 der von Flüssigkeit unbelasteten O₂-Sensoranordnung 55 von Figur 3B.

Aus einem Vergleich der Figuren 4B und 5B wird deutlich, dass mit zunehmender Flüssigkeitsbelastung der O₂-Sensoranordnung 55 ein Zustand erreicht werden könnte, bei welchem die steigende Flanke einer O₂-Partialdruckkurve im Wechselabschnitt beim Übergang von einer Exspirationsphase in eine Inspirationsphase so kurz ausfallen kann, dass ein aussagekräftiger O₂-Gradientenwert an dieser Flanke nicht mehr sicher bestimmt werden kann. Dann könnte das O₂-Gradientenkriterium drohen, zu versagen.

Im vorliegenden Fall ist auch in der flüssigkeitsbelasteten O₂-Sensoranordnung 55, die zu den Gehaltswerten der Figuren 5A und 5B führt, das Gradientenkriterium erfüllt.

Zusätzlich werden auch an der O₂-Partialdruckkurve 78" von Figur 5B, wie schon zuvor anhand der Figuren 3B und 4B erläutert, ein Inspirationsphasenabschnitt II-O2" zu Ermittlung des charakteristischen inspiratorischen O₂-Gehaltswerts 90" definiert und ein Exspirationsphasenabschnitt EI-02" zur Ermittlung des charakteristischen exspiratorischen O₂-Gehaltswerts 92" definiert.

Die Beatmungsfrequenz in den Figuren 5A und 5B ist etwas niedriger als in den vorhergehenden Figuren 3A bis 4B, weshalb die Auswertevorrichtung 15 die zeitlichen Abstände des Beginns und des Endes der Atemhub-Teilphasenabschnitte II-O2" und EI-02" von dem Bezugszeitpunkt t82" durch geringfügige Erhöhung angepasst hat. Es ist jedoch ebenso möglich, den Beginn und das Ende der Atemhub-Teilphasenabschnitte II-O2" und EI-02" so zu definieren, dass die Atemhub-Teilphasenabschnitte II-O2" und EI-02" bei allen vernünftigerweise zu erwartenden Atemfrequenzen im Betrieb der Beatmungsvorrichtung 10 in den Plateauabschnitten liegen. Die Atemhub-Teilphasenabschnitte II-O2" und EI-02" können zu diesem Zweck zeitlich kürzer als dargestellt ausfallen.

In der in Figur 5B ersten Inspirationsphase, in welcher der Inspirationsphasenabschnitt II-O2" eingezeichnet ist, ergibt sich ein über den Inspirationsphasenabschnitt II-O2" arithmetisch gemittelter O₂-Gehaltswert als der charakteristische inspiratorische Gehaltswert 90" von 156,8 mbar. Im nachfolgenden Exspirationsphasenabschnitt EI-O2" ergibt sich ein über den Exspirationsphasenabschnitt EI-O2" arithmetisch gemittelter O₂-Gehaltswert als der charakteristische exspiratorische Gehaltswert 92" von etwa 150,4 mbar. Hieraus ergibt sich ein O₂-Unterschiedswert von 6,4 mbar bzw. ein atmosphärenbezogenen O₂-Unterschiedswert von 0,006. Der Atmosphärendruck von 1013 mbar ist unverändert.

Die Auswertevorrichtung 15 vergleicht den ermittelten atmosphärenbezogenen O₂-Unterschiedswert von 0,006 mit dem oben bereits genannten O₂-Unterschiedsgrenzwert von 0,01 und stellt fest, dass der ermittelte atmosphärenbezogenen O₂-Unterschiedswert kleiner als der vorbestimmte O₂-Unterschiedsgrenzwert ist. Das Unterschiedskriterium ist somit zusätzlich zum Gradientenkriterium erfüllt. Die Auswertevorrichtung 15 gibt auch im Falle der Beatmungssituation der Figuren 5A und 5B ein eine Degradation der O₂-Sensoranordnung 55 anzeigen-des Signal aus.

Nachzutragen ist, dass die oben bezeichneten Inspirationsphasenabschnitte und Exspirationsphasenabschnitte auch zur Ermittlung charakteristischer inspiratorischer CO₂-Gehaltswerte und charakteristischer exspiratorischer CO₂-Gehaltswerte herangezogen werden können. Auch diese charakteristischen CO₂-Gehaltswerte können über ihren zugehörigen Atemhub-Teilphasenabschnitt hinweg arithmetisch gemittelte Werte sein. Auf Grundlage wenigstens eines charakteristischen exspiratorischen CO₂-Gehaltswerts und wenigstens eines charakteristischen inspiratorischen CO₂-Gehaltswerts kann ein CO₂-Unterschiedswert berechnet werden, etwa als Betrag der Differenz der charakteristischen Gehaltswerte. Auf Grundlage des so erhaltenen CO₂-Unterschiedswerts kann der oben genannte Validierungsbetrag oder/und der Validierungsanteil ermittelt werden. Beispielsweise kann der Validierungsbetrag ein vorbestimmter Anteil des CO₂-Unterschiedswerts sein. Der Validierungsanteil in Prozent kann abhängig vom CO₂-Unterschiedswert bestimmbar sein, etwa durch Hinterlegung einer Tabelle oder eines Kennfelds von Validierungsanteilen in Abhängigkeit vom CO₂-Unterschiedswert.

Auch wenn die Atemhub-Teilphasenabschnitte zur Bestimmung charakteristischer Gehaltswerte von O₂ und CO₂ die gleichen sein können, soll nicht ausgeschlossen sein, dass für jeden Beatmungsgasbestandteil: O₂ und CO₂, verschiedene Atemhub-Teilphasenabschnitte bestimmt werden, etwa aufgrund von unterschiedlichen vorbestimmten Zeitabständen des Beginns und des Endes eines Atemhub-Teilphasenabschnitts von einem Bezugszeitpunkt, wie dem Zeitpunkt des Auftretens der betragsmäßig größten zeitlichen Änderung des CO₂-Gehaltswerts. Der Bezugszeitpunkt ist bevorzugt in einem Wechselabschnitt im Übergang von einer Exspirationsphase zu einer Inspirationsphase gelegen.

Das in der vorliegenden Anmeldung zu der Bestimmung von charakteristischen inspiratorischen und exspiratorischen O₂-Gehaltswerten Gesagte gilt mutatis mutandis auch für charakteristische inspiratorische und exspiratorische CO₂-Gehaltswerte mit der Maßgabe, dass O₂ durch CO₂ zu ersetzen ist.

Es ist klar, dass die Prüfvorgänge, die die Auswertevorrichtung an den von den Sensorsignalen übertragenen Informationen ausführt, oben lediglich beispielhaft anhand von grafischen Darstellungen dieser Informationen erläutert wurde. Die Prüfvorgänge werden jedoch an Zahlenwerten bzw. Zahlenwertepaaren oder allgemein an verknüpften Zahlenwerten ausgeführt.

Einem in der vorliegenden Anmeldung angesprochenen Gehaltswert steht jeder mit dem Gehaltswert in eindeutigem funktionellem Zusammenhang stehende Wert gleich.

Die vorliegende Erfindung gestattet, eine Degradation der O₂-Sensoranordnung 55 ohne zusätzliche Sensoren rechtzeitig zu erkennen.

## Patentansprüche

1. Beatmungsvorrichtung (10) zur künstlichen Beatmung mit
- einer Beatmungsgasquelle (12),
- einer Beatmungsleitungsanordnung (20), um inspiratorisches Beatmungsgas von der Beatmungsgasquelle (12) zu einer patientenseitigen, proximalen Beatmungsgas-Auslassöffnung (62) zu leiten und um exspiratorisches Beatmungsgas von einer proximalen Beatmungsgas-Einlassöffnung (62) weg zu leiten,
- einer Druckveränderungsanordnung (16) zur Veränderung des Drucks des in der Beatmungsleitungsanordnung (20) strömenden Beatmungsgases,
- einer Steuervorrichtung (14), welche dazu ausgebildet ist, den Betrieb der Beatmungsgasquelle (12) oder/und der Druckveränderungsanordnung (16) zu steuern,
- einer Auswertevorrichtung (15) zur Verarbeitung von Sensorsignalen, und
- einer O₂-Sensoranordnung (55) zur Ermittlung eines einen Sauerstoffgehalt des in der Beatmungsleitungsanordnung (20) strömenden Beatmungsgases repräsentierenden O₂-Gehaltswerts (78; 78'; 78"), wobei die O₂-Sensoranordnung (55) O₂-Sensorsignale, welche Information über den O₂-Gehaltswert (78; 78'; 78") enthalten, an die Auswertevorrichtung (15) ausgibt,
**dadurch gekennzeichnet, dass** die Auswertevorrichtung (15) dazu ausgebildet ist, auf Grundlage der O₂-Sensorsignale einen eine Änderung des O₂-Gehaltswerts (78; 78'; 78") repräsentierenden O₂-Änderungswert zu bestimmen und dann, wenn der O₂-Änderungswert eine vorbestimmte Bedingung erfüllt, auf eine Degradation der O₂-Sensoranordnung (55) zu schließen und ein Signal auszugeben.

2. Beatmungsvorrichtung (10) nach Anspruch 1,
**dadurch gekennzeichnet, dass** die Auswertevorrichtung (15) dazu ausgebildet ist, das Signal nur dann auszugeben, wenn die vorbestimmte Bedingung eine vorbestimmte Mehrzahl von Malen innerhalb einer vorbestimmten Mehrzahl von Atemhüben erfüllt ist.

3. Beatmungsvorrichtung (10) nach Anspruch 1 oder 2,
**dadurch gekennzeichnet, dass** die Auswertevorrichtung (15) dazu ausgebildet ist, als den O₂-Änderungswert einen O₂-Unterschiedswert zu ermitteln, welcher einen betragsmäßigen Unterschied zwischen einem charakteristischen exspiratorischen O₂-Gehaltswert (92; 92'; 92") des exspiratorischen Beatmungsgases und einem charakteristischen inspiratorischen O₂-Gehaltswert (90; 90'; 90") des inspiratorischen Beatmungsgases repräsentiert, wobei die vorbestimmte Bedingung ist, dass der O₂-Unterschiedswert in einer vorbestimmten relativen Beziehung zu einem O₂-Unterschiedsgrenzwert steht.

4. Beatmungsvorrichtung (10) nach Anspruch 3,
**dadurch gekennzeichnet, dass** die Auswertevorrichtung (15) dazu ausgebildet ist, bei einer Durchführung der Vergleiche von O₂-Unterschiedswerten mit dem O₂-Unterschiedsgrenzwert wenigstens einen Atmosphärenzustandswert zu berücksichtigen, welcher einen Zustand der Umgebungsatmosphäre der Beatmungsvorrichtung (10) repräsentiert.

5. Beatmungsvorrichtung (10) nach Anspruch 4,
**dadurch gekennzeichnet, dass** die Auswertevorrichtung (15) entweder dazu ausgebildet ist, den O₂-Unterschiedsgrenzwert in Abhängigkeit von dem wenigstens einen Atmosphärenzustandswert zu ermitteln,
oder dazu ausgebildet ist, aus dem O₂-Unterschiedswert und dem wenigstens einen Atmosphärenzustandswert einen atmosphärenbezogenen O₂-Unterschiedswert zu ermitteln.

6. Beatmungsvorrichtung (10) nach Anspruch 4 oder 5,
**dadurch gekennzeichnet, dass** der wenigstens eine Atmosphärenzustandswert den Atmosphärendruck repräsentiert, dass der O₂-Unterschiedswert einen O₂-Partialdruck-Unterschiedswert zwischen einem O₂-Partialdruck in dem exspiratorischen Beatmungsgas und einem O₂-Partialdruck in dem inspiratorischen Beatmungsgas repräsentiert.

7. Beatmungsvorrichtung (10) nach einem der vorhergehenden Ansprüche, unter Einbeziehung des Anspruchs 3,
**dadurch gekennzeichnet, dass** der charakteristische inspiratorische O₂-Gehaltswert (90; 90'; 90") einem Inspirationsphasenabschnitt (II-O₂, II-O₂') entstammt, welcher einen ersten zeitlichen Abstand vom Beginn einer Inspirationsphase und einen zweiten zeitlichen Abstand vom Ende der Inspirationsphase entfernt gelegen ist, oder/und dass der charakteristische exspiratorische O₂-Gehaltswert (92; 92'; 92") einem Exspirationsphasenabschnitt (EI-O₂, EI-O₂') entstammt, welcher einen dritten zeitlichen Abstand vom Beginn einer Exspirationsphase und einen vierten zeitlichen Abstand vom Ende der Exspirationsphase entfernt gelegen ist, wobei bevorzugt die Auswertevorrichtung (15) dazu ausgebildet ist, den Inspirationsphasenabschnitt (II-O₂, II-O₂') oder/und den Exspirationsphasenabschnitt (EI-O₂, EI-O₂') zu identifizieren und darin einen charakteristischen inspiratorischen O₂-Gehaltswert (90; 90'; 90") bzw. einen charakteristischen exspiratorischen O₂-Gehaltswert (92; 92'; 92") zu ermitteln.

8. Beatmungsvorrichtung (10) nach einem der vorhergehenden Ansprüche,
**dadurch gekennzeichnet, dass** die Beatmungsvorrichtung (10) zusätzlich eine CO₂-Sensoranordnung (54) aufweist, welche zur Ermittlung eines einen Kohlenstoffdioxidgehalt des in der Beatmungsleitungsanordnung (20) strömenden Beatmungsgases repräsentierenden CO₂-Gehaltswerts (76; 76'; 76") ausgebildet ist, wobei die CO₂-Sensoranordnung (54) CO₂-Sensorsignale, welche Information über den CO₂-Gehaltswert (76; 76'; 76") enthalten, an die Auswertevorrichtung (15) ausgibt.

9. Beatmungsvorrichtung (10) nach Anspruch 8,
**dadurch gekennzeichnet, dass** die Auswertevorrichtung (15) dazu ausgebildet ist, aus den CO₂-Sensorsignalen einen eine zeitliche Änderung des CO₂-Gehaltswerts (76; 76'; 76") im exspiratorischen Beatmungsgas repräsentierenden CO₂-Gradientenwert zu ermitteln, und dass die Auswertevorrichtung (15) weiter dazu ausgebildet ist, als den O₂-Änderungswert einen eine zeitliche Änderung des O₂-Gehaltswerts (78; 78'; 78") im inspiratorischen Beatmungsgas repräsentierenden O₂-Gradientenwert zu ermitteln, wobei die vorbestimmte Bedingung ist, dass ein Verhältnis des CO₂-Gradientenwerts zu dem O₂-Gradientenwert in einer vorbestimmten relativen Beziehung zu einem Änderungsverhältnisgrenzwert steht.

10. Beatmungsvorrichtung (10) nach Anspruch 9,
**dadurch gekennzeichnet, dass** die Auswertevorrichtung (15) dazu ausgebildet ist, auf Grundlage einer Mehrzahl von CO₂-Sensorsignalen eines Atemhubs die betragsmäßig größte zeitlichen Änderung des CO₂-Gehaltswerts (76; 76'; 76") hin zu kleiner werdenden Werten als den CO₂-Gradientenwert zu ermitteln, wobei bevorzugt die Auswertevorrichtung (15) dazu ausgebildet ist, den Zeitpunkt (t82, t82') des Auftretens des CO₂-Gradientenwerts als Bezugszeitpunkt zu verwenden und auf Grundlage einer Mehrzahl von O₂-Sensorsignalen eines Atemhubs einen Änderungswert des O₂-Gehaltswerts (78; 78'; 78") als den O₂-Gradientenwert zu verwenden, welcher in einem vorbestimmten zeitlichen Abstand vom CO₂-Gradientenwert auftritt, wobei weiter bevorzugt der vorbestimmte zeitliche Abstand derart gewählt ist, dass der O₂-Gradientenwert in einem Abschnitt eines Wechsels von einer Exspirationsphase zu einer Inspirationsphase mit mit zeitlichem Fortschritt steigenden Sauerstoffgehalten gelegen ist, wobei noch weiter bevorzugt der CO₂-Gradientenwert in einem Abschnitt eines Wechsels von einer Exspirationsphase zu einer Inspirationsphase gelegen ist, wobei der vorbestimmte zeitliche Abstand in einem Bereich von 25 bis 80 ms, vorzugsweise in einem Bereich von 35 bis 65 ms, besonders bevorzugt in einem Bereich von 45 bis 55 ms, liegt, sodass der O₂-Gradientenwert bevorzugt in einem Inspirationsvorgang gelegen ist, welcher unmittelbar auf den Exspirationsvorgang folgt, in dem der CO₂-Gradientenwert liegt.

11. Beatmungsvorrichtung (10) nach einem der Ansprüche 8 bis 10, unter Einbeziehung des Anspruchs 7,
**dadurch gekennzeichnet, dass** die Auswertevorrichtung (15) dazu ausgebildet ist, den Inspirationsphasenabschnitt (II-O₂, II-O₂') oder/und den Exspirationsphasenabschnitt (EI-O₂, EI-O₂') zu identifizieren und darin einen charakteristischen inspiratorischen O₂-Gehaltswert (90; 90'; 90") bzw. einen charakteristischen exspiratorischen O₂-Gehaltswert (92; 92'; 92") zu ermitteln, wobei die Auswertevorrichtung (15) dazu ausgebildet ist, auf Grundlage einer Mehrzahl von CO₂-Sensorsignalen eines Atemhubs die betragsmäßig größte zeitliche Änderung des CO₂-Gehaltswerts (76; 76'; 76") hin zu kleiner werdenden Werten zu ermitteln, wobei die Auswertevorrichtung (15) weiter dazu ausgebildet ist, den Inspirationsphasenabschnitt (II-O₂, II-O₂') oder/und den Exspirationsphasenabschnitt (EI-O₂, EI-O₂') in Abhängigkeit von der zeitlichen Bestimmung des Auftretens der betragsmäßig größten zeitlichen Änderung des CO₂-Gehaltswerts (76; 76'; 76") hin zu kleiner werdenden Werten zu identifizieren.

12. Beatmungsvorrichtung (10) nach einem der Ansprüche 8 bis 11, unter Einbeziehung des Anspruchs 7,
**dadurch gekennzeichnet, dass** die Auswertevorrichtung (15) den charakteristischen inspiratorischen O₂-Gehaltswert (90; 90'; 90") als einen Mittelwert über eine Mehrzahl von O₂-Sensorsignalen im Inspirationsphasenabschnitt (II-O₂, II-O₂') bildet oder/und dass die Auswertevorrichtung (15) den charakteristischen exspiratorischen O₂-Gehaltswert (92; 92'; 92") als einen Mittelwert über eine Mehrzahl von O₂-Sensorsignalen im Exspirationsphasenabschnitt (II-O₂, II-O₂') bildet.

13. Beatmungsvorrichtung (10) nach einem der vorhergehenden Ansprüche, unter Einbeziehung wenigstens eines der Ansprüche 10 oder 11,
**dadurch gekennzeichnet, dass** die Auswertevorrichtung (15) dazu ausgebildet ist, in einer Phase eines Wechsels von einem Exspirationsvorgang zu einem Inspirationsvorgang ein Untersuchungszeitintervall (ZI) zu identifizieren und nur innerhalb des Untersuchungszeitintervalls (ZI) nach einem Auftreten der betragsmäßig größten zeitlichen Änderung des CO₂-Gehaltswerts (76; 76'; 76") hin zu kleiner werdenden Werten zu suchen.

14. Beatmungsvorrichtung (10) nach Anspruch 13, unter Einbeziehung des Anspruchs 8,
**dadurch gekennzeichnet, dass** die Auswertevorrichtung (15) zur Identifikation des Untersuchungszeitintervalls (ZI) aus einer Mehrzahl an CO₂-Sensorsignalen als ein Auslöseereignis bestimmt, wann der CO₂-Gehaltswert (76; 76'; 76") einen vorbestimmten CO₂-Auslösegrenzwert (79) unterschreitet, und ausgehend von dem ermittelten Auslösezeitereignis ein Zeitintervall als ein Kandidatenintervall (KI) definiert, wobei bevorzugt die Auswertevorrichtung (15) dazu ausgebildet ist, das Kandidatenintervall (KI) dann als das Untersuchungszeitintervall (ZI) zu verwenden, wenn die Auswertevorrichtung (15) in einem Validierungsvorgang ermittelt, dass in dem Kandidatenintervall (KI) ein CO₂-Gehaltswerteniveau, welches während einer Exspirationsphase, die wenigstens den Beginn des Kandidatenintervalls (KI) enthält, oder welches zu Beginn des Kandidatenintervalls (KI) vorherrscht, um wenigstens einen vorbestimmten Validierungsbetrag (VB) oder/und um wenigstens einen vorbestimmten Validierungsanteil abnimmt, wobei weiter bevorzugt die Auswertevorrichtung (15) dazu ausgebildet ist, den Validierungsvorgang ab dem Auslöseereignis iterativ mit einem jeweils modifizierten Kandidatenintervall (KI) auszuführen.

15. Beatmungsvorrichtung (10) nach Anspruch 14,
**dadurch gekennzeichnet, dass** die Auswertevorrichtung (15) dazu ausgebildet ist, den vorbestimmten Validierungsbetrag (VB) oder/und den vorbestimmten Validierungsanteil auf Grundlage zurückliegender CO₂-Sensorsignale zu ermitteln.

## Claims

1. Ventilation device (10) for artificial respiration with
- A respiratory gas source (12),
- A ventilation line arrangement (20) in order to lead inspiratory respiratory gas from the respiratory gas source (12) to a patient-side proximal respiratory gas outlet aperture (62) and in order to lead expiratory respiratory gas away from a proximal respiratory gas inlet aperture (62),
- A pressure modification arrangement (16) for modifying the pressure of the respiratory gas flowing in the ventilation line arrangement (20),
- A control device (14) designed to control the operation of the respiratory gas source (12) and/or of the pressure modification arrangement (16),
- An evaluation device (15) for processing sensor signals, and
- An O₂ sensor assembly (55) for ascertaining an O₂ content value (78; 78'; 78") which represents an oxygen content of the respiratory gas flowing in the ventilation line arrangement (20), where the O₂ sensor assembly (55) outputs to the evaluation device (15) O₂ sensor signals which contain information about the O₂ content value (78; 78'; 78"),
**characterized in that** the evaluation device (15) is designed to determine on the basis of the O₂ sensor signals an O₂ change value which represents a change in the O₂ content value (78; 78'; 78") and when the O₂ change value satisfies a predetermined condition to deduce a degradation of the O₂ sensor assembly (55) and output a signal.

2. Ventilation device (10) according to Claim 1,
**characterized in that** the evaluation device (15) is designed to output the signal only if the predetermined condition is satisfied a predetermined plurality of times within a predetermined plurality of breaths.

3. Ventilation device (10) according to Claim 1 or 2,
**characterized in that** the evaluation device (15) is designed to ascertain, as the O₂ change value, an O₂ difference value which represents a quantitative difference between a characteristic expiratory O₂ content value (92; 92'; 92") of the expiratory respiratory gas and a characteristic inspiratory O₂ content value (90; 90'; 90") of the inspiratory respiratory gas, where the predetermined condition is that the O₂ difference value is related in a predetermined relative relationship to an O₂ difference limit.

4. Ventilation device (10) according to Claim 3,
**characterized in that** the evaluation device (15) is designed to take into account, when performing the comparisons of O₂ difference values with the O₂ difference limit, at least one atmospheric state value which represents a state of the ambient atmosphere of the ventilation device (10).

5. Ventilation device (10) according to Claim 4,
**characterized in that** the evaluation device (15) is either designed to ascertain the O₂ difference limit as a function of the at least one atmospheric state value,
or is designed to ascertain from the O₂ difference value and the at least one atmospheric state value an atmosphere-based O₂ difference value.

6. Ventilation device (10) according to Claim 4 or 5,
**characterized in that** the at least one atmospheric state value represents the atmospheric pressure, that the O₂ difference value represents an O₂ partial pressure difference value between an O₂ partial pressure in the expiratory respiratory gas and an O₂ partial pressure in the inspiratory respiratory gas.

7. Ventilation device (10) according to one of the preceding Claims, by reference to Claim 3,
**characterized in that** the characteristic inspiratory O₂ content value (90; 90'; 90") derives from an inspiratory phase segment (II-O₂, II-O₂') which is located at a first temporal distance from the beginning of an inspiratory phase and a second temporal distance from the end of the inspiratory phase, and/or that the characteristic expiratory O₂ content value (92; 92'; 92") derives from an expiratory phase segment (EI-O₂, EI-O₂') which is located at a third temporal distance from the beginning of an expiratory phase and at a fourth temporal distance from the end of the expiratory phase,
wherein the evaluation device (15) is preferably designed to identify the inspiratory phase segment (II-O₂, II-O₂') and/or the expiratory phase segment (EI-O₂, EI-O₂') and to ascertain therein a characteristic inspiratory O₂ content value (90; 90'; 90") and/or a characteristic expiratory O₂ content value (92; 92'; 92"), respectively.

8. Ventilation device (10) according to one of the preceding Claims,
**characterized in that** the ventilation device (10) additionally exhibits a CO₂ sensor assembly (54) which is designed to ascertain a CO₂ content value (76; 76'; 76") representing a carbon dioxide content of the respiratory gas flowing in the ventilation line arrangement (30), where the CO₂ sensor assembly (54) outputs to the evaluation device (15) CO₂ sensor signals which contain information about the CO₂ content value (76; 76'; 76").

9. Ventilation device (10) according to Claim 8,
**characterized in that** the evaluation device (15) is designed to ascertain from the CO₂ sensor signals a CO₂ gradient value representing a temporal change in the CO₂ content value (76; 76'; 76") in the expiratory respiratory gas, and that the evaluation device (15) is further designed to ascertain, as the O₂ change value, an O₂ gradient value representing a temporal change in the O₂ content value (78; 78'; 78") in the inspiratory respiratory gas, where the predetermined condition is that a ratio of the CO₂ gradient value to the O₂ gradient value is related in a predetermined relative relationship to a change ratio limit.

10. Ventilation device (10) according to Claim 9,
**characterized in that** the evaluation device (15) is designed to ascertain, on the basis of a plurality of CO₂ sensor signals of a breath, the quantitatively largest temporal change in the CO₂ content value (76; 76'; 76") towards decreasing values as the CO₂ gradient value,
wherein preferably the evaluation device (15) is designed to use the point in time (t82, t82') of the occurrence of the CO₂ gradient value as the reference point in time and on the basis of a plurality of O₂ sensor signals of a breath to use a change value of the O₂ content value (78; 78'; 78") as the O₂ gradient value which occurs at a predetermined temporal distance from the CO₂ gradient value,
wherein the predetermined temporal distance is further preferably chosen in such a way that the O₂ gradient value lies in a segment of a changeover from an expiratory phase to an inspiratory phase with the oxygen contents increasing with time,
wherein the CO₂ gradient value lies even more preferably in a segment of a changeover from an expiratory phase to an inspiratory phase, where the predetermined temporal distance lies in a range from 25 to 80 ms, preferably in a range from 35 to 65 ms, especially preferably in a range from 45 to 55 ms, such that the O₂ gradient value preferably lies in an inspiratory process which immediately follows the expiratory process in which the CO₂ gradient value lies.

11. Ventilation device (10) according to one of the Claims 8 to 10, by reference to Claim 7,
**characterized in that** the evaluation device (15) is configured to identify the inspiratory phase segment (II-O₂, II-O₂') and/or the expiratory phase segment (EI-O₂, EI-O₂') and to ascertain therein a characteristic inspiratory O₂ content value or an expiratory O₂ content value, respectively, wherein the evaluation device (15) is configured to ascertain, on the basis of a plurality of CO₂ sensor signals of a breath, the quantitatively largest temporal change in the CO₂ content value (76; 76'; 76") towards decreasing values, where the evaluation device (15) is further configured to identify the inspiratory phase segment (II-O₂, II-O₂') and/or the expiratory phase segment (EI-O₂, EI-O₂') as a function of the temporal determination of the occurrence of the quantitatively largest temporal change in the CO₂ content value (76; 76'; 76") towards decreasing values.

12. Ventilation device (10) according to one of the Claims 9 to 11, by reference to Claim 7,
**characterized in that** the evaluation device (15) forms the characteristic inspiratory O₂ content value (90; 90'; 90") as an average over a plurality of O₂ sensor signals in the inspiratory phase segment (II-O₂, II-O₂') and/or that the evaluation device (15) forms the characteristic expiratory O₂ content value (92; 92'; 92") as an average over a plurality of O₂ sensor signals in the expiratory phase segment (II-O₂, II-O₂').

13. Ventilation device (10) according to one of the preceding Claims, by reference to at least one of the Claims 10 or 11,
**characterized in that** the evaluation device (15) is designed to identify an examination time interval (ZI) in a phase of a changeover from an expiratory process to an inspiratory process and to search only within the examination time interval (ZI) for an occurrence of the quantitatively largest temporal change in the CO₂ content value (76; 76'; 76") towards decreasing values.

14. Ventilation device (10) according to Claim 13, by reference to Claim 8,
**characterized in that** the evaluation device (15), in order to identify the examination time interval (ZI) from a plurality of CO₂ sensor signals as a triggering event, determines when the CO₂ content value (76; 76'; 76") falls below a predetermined CO₂ triggering limit (79), and starting from the ascertained triggering time event defines a time interval as a candidate interval (KI),
wherein preferably the evaluation device (15) is designed to use the candidate interval (KI) as the examination time interval (ZI), when the evaluation device (15) ascertains in a validation procedure that during the candidate interval (KI) a CO₂ content value level which during an expiratory phase which includes at least the beginning of the candidate interval (KI), or which prevails at the beginning of the candidate interval (KI), decreases by at least a predetermined validation magnitude (VB) and/or by at least a predetermined validation - fraction,
wherein further preferably the evaluation device (15) is designed to perform the validation procedure iteratively as from the triggering event, in each case with a modified candidate interval (KI).

15. Ventilation device (10) according to claim 14,
**characterized in that** the evaluation device (15) is designed to ascertain the predetermined validation magnitude (VB) and/or the predetermined validation fraction on the basis of preceding CO₂ sensor signals.

## Revendications

1. Dispositif de ventilation (10) pour la respiration artificielle avec
- une source de gaz respiratoire (12),
- un agencement de conduit de ventilation (20) pour diriger le gaz de ventilation inspiratoire de la source de gaz respiratoire (12) vers un orifice de sortie de gaz respiratoire proximal (62) du côté du patient et pour diriger le gaz de ventilation expiratoire à l'écart d'un orifice d'entrée de gaz respiratoire proximal (62),
- un agencement de modification de pression (16) pour modifier la pression du gaz respiratoire s'écoulant dans l'agencement de conduit de ventilation (20),
- un dispositif de commande (14) qui est conçu pour commander le fonctionnement de la source de gaz respiratoire (12) ou/et de l'agencement de modification de pression (16),
- un dispositif d'évaluation (15) pour le traitement des signaux de capteurs, et
- un agencement de capteurs d'O₂ (55) pour déterminer une valeur de teneur en O₂ (78; 78'; 78") représentant une teneur en oxygène du gaz respiratoire circulant dans l'agencement de conduit de ventilation (20), dans lequel l'agencement de capteurs d'O₂ (55) délivre au dispositif d'évaluation (15) des signaux de capteurs d'O₂ qui contiennent des informations sur la valeur de teneur en O₂ (78; 78'; 78"),
**caractérisé en ce que** le dispositif d'évaluation (15) est conçu pour déterminer, sur la base des signaux de capteur d'O₂, une valeur de variation d'O₂ représentant une variation de la valeur de teneur en O₂ (78; 78'; 78") et, ensuite, si la valeur de variation d'O₂ remplit une condition prédéterminée, pour conclure à une dégradation de l'agencement de capteur d'O₂ (55) et pour émettre un signal.

2. Dispositif de ventilation (10) selon la revendication 1,
**caractérisé en ce que** le dispositif d'évaluation (15) est conçu pour émettre le signal uniquement lorsque la condition prédéterminée est remplie une pluralité prédéterminée de fois au cours d'une pluralité prédéterminée de courses respiratoires.

3. Dispositif de ventilation (10) selon la revendication 1 ou 2,
**caractérisé en ce que** le dispositif d'évaluation (15) est conçu pour déterminer, en tant que valeur de variation d'O₂, une valeur de différence d'O₂ qui représente une différence de valeur entre une valeur de teneur en O₂ expiratoire caractéristique (92; 92'; 92") du gaz de ventilation expiratoire et une valeur de teneur en O₂ inspiratoire caractéristique (90, 90'; 90") du gaz de ventilation inspiratoire, dans lequel la condition prédéterminée est que la valeur de différence d'O₂ soit dans une relation relative prédéterminée avec une valeur de différence limite de sous-débit d'O₂.

4. Dispositif de ventilation (10) selon la revendication 3,
**caractérisé en ce que** le dispositif d'évaluation (15) est configuré pour prendre en compte, lors de la réalisation des comparaisons des valeurs de différence d'O₂ avec la valeur de différence limite d'O₂, au moins une valeur d'état atmosphérique représentant un état de l'atmosphère ambiante du dispositif de ventilation (10).

5. Dispositif de ventilation (10) selon la revendication 4,
**caractérisé en ce que** le dispositif d'évaluation (15) est conçu soit pour déterminer la valeur de différence limite d'O₂ en fonction de ladite au moins une valeur d'état atmosphérique, soit pour déterminer une valeur de différence d'O₂ relative à l'atmosphère à partir de la valeur de différence d'O₂ et de ladite au moins une valeur d'état atmosphérique.

6. Dispositif de ventilation (10) selon la revendication 4 ou 5,
**caractérisé en ce que** ladite au moins une valeur d'état atmosphérique représente la pression atmosphérique, **en ce que** la valeur de différence d'O₂ représente une valeur de différence de pression partielle d'O₂ entre une pression partielle d'O₂ dans le gaz de ventilation expiratoire et une pression partielle d'O₂ dans le gaz de ventilation inspiratoire.

7. Dispositif de ventilation (10) selon l'une des revendications précédentes, en tenant compte de la revendication 3,
**caractérisé en ce que** la valeur caractéristique de teneur en O₂ inspiratoire (90; 90'; 90") provient d'une portion de phase inspiratoire (II-O₂, II-O₂') située à une première distance temporelle du début d'une phase inspiratoire et à une deuxième distance temporelle de la fin de la phase inspiratoire, ou/et **en ce que** la valeur caractéristique de teneur en O₂ expiratoire (92; 92'; 92") provient d'une portion de phase expiratoire (EI-O₂, EI-O₂') qui est située à une troisième distance temporelle du début d'une phase expiratoire et à une quatrième distance temporelle de la fin de la phase expiratoire, dans lequel le dispositif d'évaluation (15) est de préférence conçu pour identifier la portion de phase inspiratoire (EI-O₂, EI-O₂') ou/et la portion de phase expiratoire (EI-O₂, EI-O₂') et y calculer une valeur de teneur en O₂ inspiratoire caractéristique (90; 90'; 90") et respectivement une valeur de teneur en O₂ expiratoire caractéristique (92; 92'; 92").

8. Dispositif de ventilation (10) selon l'une des revendications précédentes,
**caractérisé en ce que** le dispositif de ventilation (10) présente en outre un agencement de capteurs de CO₂ (54) qui est destiné à déterminer une valeur de teneur en CO₂ (76; 76'; 76") représentant une teneur en dioxyde de carbone d'écoulant dans l'agencement de conduit de ventilation (20), dans lequel l'agencement de capteurs de CO₂ (54) délivre au dispositif d'évaluation (15) des signaux de capteurs de CO₂ qui contiennent des informations sur la valeur de teneur en CO₂ (76; 76'; 76").

9. Dispositif de ventilation 10) selon la revendication 8,
**caractérisé en ce que** le dispositif d'évaluation (15) est conçu pour déterminer, à partir des signaux de capteur de CO₂, une valeur de gradient de CO₂ représentant une variation dans le temps de la valeur de teneur en CO₂ (76; 76'; 76") dans le gaz de ventilation expiratoire, et **en ce que** le dispositif d'évaluation (15) est en outre conçu pour déterminer, en tant que valeur de variation d'O₂, une valeur de gradient de CO₂ représentant une variation dans le temps de la valeur de teneur en O₂ (78; 78'; 78") dans le gaz de ventilation inspiratoire, dans lequel la condition prédéterminée est qu'un rapport de la valeur de gradient de CO₂ à la valeur de gradient de O₂ se trouve dans une relation relative prédéterminée avec une valeur limite du rapport de variation.

10. Dispositif de ventilation (10) selon la revendication 9,
**caractérisé en ce que** le dispositif d'évaluation (15) est conçu pour déterminer, sur la base d'une pluralité de signaux de capteur de CO₂ d'une course respiratoire, la plus grande variation dans le temps de la valeur de teneur en CO₂ (76; 76'; 76") vers des valeurs plus petites que la valeur de gradient de CO₂, dans lequel le dispositif d'évaluation (15) est de préférence conçu pour utiliser l'instant (t82, t82') de l'apparition de la valeur de gradient de CO₂ comme instant de référence et pour déterminer, sur la base d'une pluralité de signaux de capteur d'O₂ d'une course respiratoire, une valeur de modification de la valeur de teneur en O₂ (78; 78'; 78") comme valeur de gradient d'O₂, qui apparaît à un intervalle de temps prédéterminé de la valeur de gradient de CO₂, dans lequel de préférence l'intervalle de temps prédéterminé est choisi de telle sorte que la valeur de gradient d'O₂ se situe dans une portion d'un passage d'une phase d'expiration à une phase d'inspiration avec des teneurs en oxygène qui augmentent avec le temps, dans lequel de préférence encore, la valeur de gradient de CO₂ est située dans une portion d'un passage d'une phase d'expiration à une phase d'inspiration, dans lequel l'intervalle de temps prédéterminé est situé dans une plage de 25 à 80 ms, de préférence dans une plage de 35 à 65 ms, de manière particulièrement préférée dans une plage de 45 à 55 ms, de sorte que la valeur du gradient d'O₂ est située de préférence dans un processus d'inspiration qui suit immédiatement le processus d'expiration dans lequel se trouve la valeur de gradient de CO₂.

11. Dispositif de ventilation (10) selon l'une des revendications 8 à 10, en tenant compte de la revendication 7,
**caractérisé en ce que** le dispositif d'évaluation (15) est configuré pour identifier la portion de phase inspiratoire (II-O₂, II-O₂') ou/et la portion de phase expiratoire (EI-O₂, EI-O₂') et pour y déterminer une valeur de teneur en O₂ inspiratoire caractéristique (90; 90'; 90") ou/et une valeur de teneur en O₂ expiratoire caractéristique (92; 92'; 92"), dans lequel le dispositif d'évaluation (15) est conçu pour déterminer, sur la base d'une pluralité de signaux de capteurs de CO₂ d'une course respiratoire, la plus grande variation dans le temps de la valeur de teneur en CO₂ (76; 76'; 76") vers des valeurs décroissantes, le dispositif d'évaluation (15) est en outre conçu pour identifier la portion de phase inspiratoire (II- O₂, II- O₂') et/ou la portion de phase expiratoire (El- O₂, El- O₂') en fonction de la détermination temporelle de l'apparition de la plus grande variation dans le temps de la valeur de teneur en CO₂ (76; 76'; 76") vers des valeurs décroissantes.

12. Dispositif de ventilation (10) selon l'une des revendications 8 à 11, en tenant compte de la revendication 7,
**caractérisé en ce que** le dispositif d'évaluation (15) détermine la valeur inspiratoire caractéristique de la teneur en O₂ (90; 90'; 90") comme valeur moyenne sur une pluralité de signaux de capteur O₂ dans la portion de phase inspiratoire (II-O₂, II-O₂') ou/et que le dispositif d'évaluation (15) forme la valeur de teneur en O₂ expiratoire caractéristique (92; 92'; 92") comme valeur moyenne sur une pluralité de signaux de capteur O₂ dans la portion de phase expiratoire (II-O₂, II-O₂').

13. Dispositif de ventilation (10) selon l'une quelconque des revendications précédentes, en tenant compte d'au moins l'une des revendications 10 ou 11, **caractérisé en ce que** le dispositif d'évaluation (15) est conçu pour identifier un intervalle temporelle d'évaluation (ZI) dans une phase de passage d'un processus expiratoire à un processus inspiratoire et pour rechercher, uniquement à l'intérieur de l'intervalle temporelle d'évaluation (ZI), une occurrence de la variation dans le temps la plus importante en valeur absolue de la valeur de teneur en CO₂ (76; 76'; 76") vers des valeurs décroissantes.

14. Dispositif de ventilation (10) selon la revendication 13, en tenant compte de la revendication 8,
**caractérisé en ce que** le dispositif d'évaluation (15) détermine, pour l'identification de l'intervalle temporelle d'évaluation (ZI), à partir d'une pluralité de signaux de capteur de CO₂ en tant qu'événement déclencheur, le moment où la valeur de teneur en CO₂ (76; 76'; 76") passe en dessous d'une valeur limite de déclenchement de CO₂ (79) prédéterminée, et définit, à partir de l'événement de temps de déclenchement déterminé, un intervalle de temps en tant qu'intervalle candidat (KI), dans lequel le dispositif d'évaluation (15) est de préférence conçu pour utiliser l'intervalle candidat (KI) en tant qu'intervalle temporelle d'évaluation (ZI) lorsque le dispositif d'évaluation (15) détermine, dans un processus de validation, qu'un niveau de valeur de teneur en CO₂ est atteint dans l'intervalle candidat (KI), qui prédomine pendant une phase expiratoire, qui contient au moins le début de l'intervalle candidat (Kl), ou qui prédomine au début de l'intervalle candidat (KI), diminue d'au moins une valeur de validation prédéterminée (VB) et/ou d'au moins une proportion de validation prédéterminée, dans lequel encore de préférence le dispositif d'évaluation (15) est conçu pour exécuter le processus de validation à partir de l'événement déclencheur de manière itérative avec un intervalle candidat (KI) respectivement modifié.

15. Dispositif de ventilation (10) selon la revendication 14,
**caractérisé en ce que** le dispositif d'évaluation (15) est conçu pour déterminer la valeur de validation prédéterminée (VB) ou/et la portion de validation prédéterminée sur la base de signaux de capteur de CO₂ antérieurs.
